# EUROPEAN PATENT SPECIFICATION

(11) **EP 3 355 940 B1**
(45) Date of publication and mention of the grant of the patent: **04.11.2026**
(21) Application number: 16784311.9
(22) Date of filing: 29.09.2016
(51) Int. Cl.: A61L 2/084

(54) **INACTIVATION OF PATHOGENS IN EX VIVO BLOOD PRODUCTS IN STORAGE BAGS USING LIGHT CENTERED ON OR AROUND 405 NM**
INAKTIVIERUNG VON PATHOGENEN IN EX VIVO BLUTPRODUKTEN IN AUFBEWAHRUNGSBEUTELN DURCH DIE VERWENDUNG VON LICHT MIT EINER WELLENLÄNGE VON ETWA 405 NM
INACTIVATION D'AGENTS PATHOGÈNES DANS DES PRODUITS SANGUINS EX VIVO DANS DES POCHES DE STOCKAGE À L'AIDE D'UNE LUMIÈRE D'UNE LONGUEUR D'ONDE DE 405 NM OU AVOIR DE 405 NM

(30) Priority: 02.10.2015 US 201562236706 P
(43) Date of publication of application: 08.08.2018
(73) Proprietor: The U.S.A. as represented by the Secretary, Department of Health and Human Services, Bethesda, MD 20852-7660 (US); The University Of Strathclyde, Glasgow G1 1XW (GB)
(72) Inventor: ATREYA, Chintamani, Silver Spring, MD 20993 (US); MACLEAN, Michelle, Glasgow G1 1XW (GB); ANDERSON, John G., Glasgow G1 1XW (GB); MACGREGOR, Scott J., Glasgow G1 1XW (GB)
(74) Representative: Forresters IP LLP
(86) International application number: PCT/US2016/054508
(87) International publication number: WO 2017/062260

(56) References cited:
- EP-A1- 0 196 515
- EP-A1- 1 693 072
- EP-A2- 0 457 196
- WO-A1-00/74731
- WO-A1-99/59645
- DE-C1- 4 209 509
- US-A1- 2004 021 089
- US-A1- 2007 009 377
- US-A1- 2010 246 169
- GIOVANNETTI RITA: "The Use of Spectrophotometry UV-Vis for the Study of Porphyrins", 1 June 2012 (2012-06-01), XP093298695, Retrieved from the Internet <URL:https://www.intechopen.com/chapters/37656> DOI: 10.5772/38797

## Description

### CROSS-REFERENCE TO RELATED APPLICATION

This application claims the benefit of U.S. provisional patent application no. 62/236,706 filed October 2, 2015.

### FIELD

This application is related to methods and devices for inactivating pathogens in ex-vivo stored blood and blood products.

### BACKGROUND

Bacterial and viral contamination of *ex vivo* stored human blood and blood components is a major complication for transfusion medicine, resulting in discarding of the products, and health risks for recipients of contaminated donor blood. Current pathogen reduction technologies (PRT) rely on the use of chemicals and/or ultraviolet light; however reported adverse events, and concerns about the PRT-treated blood components quality and toxicity, have limited the routine use of PRT worldwide.

For plasma treatment, a number of PRTs have been developed and pathogen-reduced plasma is routinely used, with several of these methods now licensed for use in North America and Europe. The original PRTs developed for plasma treatment included the use of solvent/detergent, and methylene blue in combination with visible light. More recently developed methods have employed ultraviolet (UV) light. Exposure to amotosalen (S-59) plus long-wave ultraviolet (UVA) light, and treatment with riboflavin and UV light, have been developed to treat both plasma and platelets. Many current PRTs have limitations and affect plasma coagulation factors and inhibitors of homeostasis to varying degrees, and concerns about reduced component quality and toxicity have limited or prevented the routine use of PRTs in many countries.

For platelet components, bacterial contamination still poses the highest risk of transfusion-transmitted disease despite the availability of bacterial detection methods. Although platelets usually have very low levels of bacterial contamination at the time of donation, platelets are stored at room temperature and this allows bacterial proliferation to occur throughout the storage period. Over a 5 day storage period even extremely small numbers of contaminating bacteria can multiply to very high and clinically dangerous levels.

A challenge for PRTs with platelet products is to maintain biological function of the treated cells, while achieving adequate pathogen reduction levels. For platelets this means sufficient retention of adhesive, aggregating and procoagulant properties to restore hemostasis in the recipient. Platelet treatment methods that have been developed include amotosalen/UVA treatment and riboflavin/UV-light treatment. While data of clinical and hemovigilance studies indicate that the overall safety profile of pathogen-reduced platelets is comparable to that of conventional platelets, there have been concerns over acute respiratory distress associated with UV light and photosensitizer-treated platelets.

Because of these uncertainties regarding currently available PRTs for plasma and platelet treatments, and because the full extent of future microbiological challenges cannot be predicted, methods to improve existing pathogen reduction technologies and the introduction of novel technologies remains an active area of investigation in transfusion medicine.

A first prior art arrangement is known from EP0196515A1, which relates to a method for the inactivation of viruses in compositions containing therapeutic proteins. A second prior art arrangement is known from US2010246169A1, which relates to a lighting device for inactivating medically significant bacteria.

### SUMMARY

The present invention provides an apparatus and a method for inactivating pathogens in ex vivo stored plasma and/or platelets, and use of light having a wavelength spectrum centered on or around 405 nm for deactivating pathogens in stored plasma and/or platelets, in accordance with the appended claims.

The technology disclosed herein provides a new solution for the decontamination of blood products that overcomes limitations associated with currently available PRTs. This disclosure describes, for example, the application of violet-blue light centered around 405 nm wavelengths for pathogen reduction in blood products such as plasma and platelets. Unlike previous methods that require the decontamination treatment of the blood product prior to deposition in the storage container, methods described herein permit decontamination to take place after the blood products enter the final storage receptacle, thereby minimizing any unnecessary further contamination from environmental sources during subsequent transfer operations.

Another aspect of the technology is that, unlike previous PRTs which utilize light radiation from the ultra-violet (UV) region of the spectrum, disclosed technologies utilize radiation from the visible light spectrum, such as with wavelengths particularly within the range of 380-420 nm and especially at or around 405 nm. This is beneficial as it permits inactivation of pathogens to occur without the photochemical damage to sensitive blood product components caused by high energy UV-light photons associated with UV light. In addition, visible light has longer wavelengths that can more efficiently penetrate through blood storage bags and through the opaque blood products at lower intensities and without damaging the storage bag material. Moreover it does not require the addition of chemical photosensitising agents which can also have a deleterious impact on the quality of blood products.

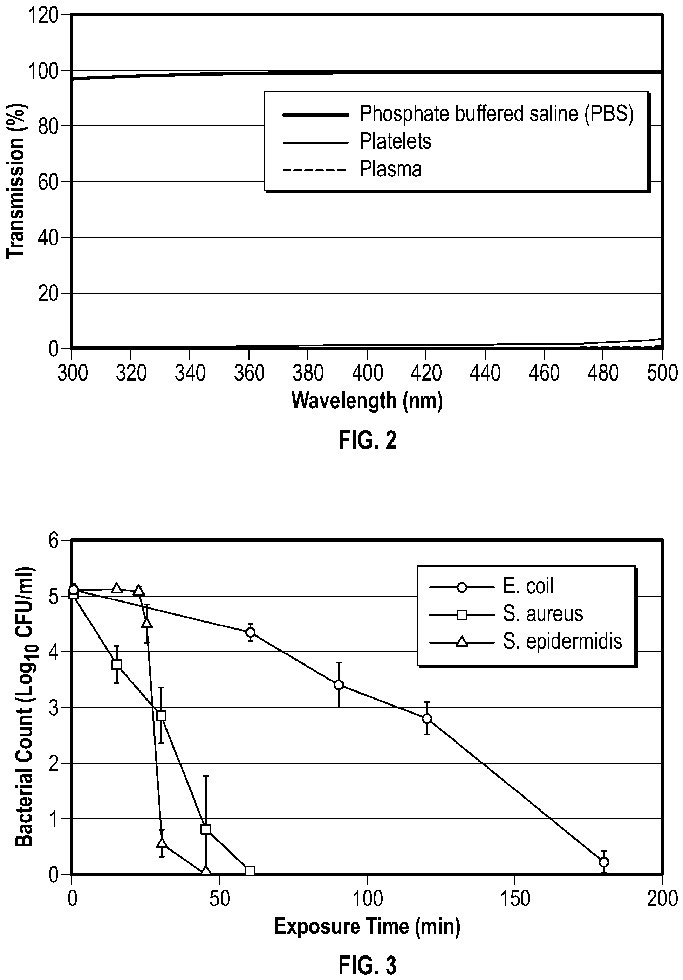

The disclosed technology makes use of properties of visible light, which have somewhat greater penetrability powers than UV light, thereby permitting the concept of in situ treatment of bagged products by enabling penetration of light through the transparent bag material and into the stored product. The low transmissibility property of blood plasma and platelets is shown in Figure 2.

One aspect of the disclosed technology is to combine the use of a sufficient level of irradiation of visible light into the product which is mixed by agitation to a sufficient extent that the blood product circulates within the container and effective decontamination of most of or substantially the entire product volume is achieved. Agitation also prevents aggregation of platelets and other blood components.

Another aspect is the specification of the levels of irradiation needed to achieve the desired decontamination effect. For an LED light source emitting light at 405 nm with bandwidth of ~10 nm, for example, the irradiance level at the blood product surface is desirably within a certain range that inactivates pathogens but does not harm the blood products (such as at least 3 mWcm⁻² and less than 80 mWcm⁻²), with exposures ranging from minutes to hours, depending on the level of irradiance used. Use of higher irradiance levels enables faster treatment times, e.g. for rapid treatment (of the order of minutes), and use of lower irradiance levels facilitates longer treatment, e.g. for background decontamination during standard storage conditions (of the order of hours). These values can permit effective microbial decontamination of the blood product without causing any significant deleterious effects on blood product components. Hence in some embodiments, the blood products do not contain chemical photosensitization agent additives.

Also disclosed herein is an exemplary apparatus for inactivating pathogens in ex vivo stored plasma and/or platelets. The apparatus includes a treatment chamber containing at least one sealed container storing plasma and/or platelets without an added photosensitizing agent present in the plasma and/or platelets, and at least one light source operable to emit light that is directed to the plasma and/or platelets storage container in the treatment chamber. The emitted light has a wavelength or wavelength spectrum and an intensity sufficient to penetrate through a wall of the container and into the plasma and/or platelets, such that the irradiance of the light reaching the plasma and/or platelets inside the wall of the container is sufficient to inactivate pathogens in the plasma and/or platelets without the presence of an added photosensitizing agent in the plasma and/or platelets and without a detrimental effect on the plasma and/or platelets, such that the blood product is suitable for future medical use. The light emitted by the light source has a wavelength spectrum centered on or around 405nm, the center wavelength being the wavelength having the greatest spectral irradiance. The treatment chamber can include a viewing window that filters out the treatment light wavelengths and allows other visible light wavelengths to pass through so that an operator can see inside the chamber. The chamber can include reflective walls, lenses, diffusers, and/or other optical features for directing light at the blood storage containers. The containers can rest on a tray in the chamber that vibrates or otherwise agitates the blood product in the container to prevent aggregation and/or to circulate the blood product inside the container for increased radiation exposure. The blood product containers can also be suspended in the chamber or positioned in other orientations. Light sources can be positioned on different sides of the container to expose more than one surface of the container at the same time. The apparatus can include any of various other features, such as a fan, air circulation system, temperature monitoring system, sensors, and a control system that controls the light sources and other active features of the apparatus.

The foregoing and other objects, features, and advantages of the disclosed technology will become more apparent from the following detailed description, which proceeds with reference to the accompanying figures.

### BRIEF DESCRIPTION OF THE DRAWINGS

Figure 1A is a perspective view of an exemplary treatment chamber for inactivating pathogens in stored blood products.
Figure 1B is a schematic view of an exemplary treatment chamber having various light sources, a tray, and a blood product storage container on the tray being irradiated with light from the light sources.
Figure 1C shows an exemplary blood product storage container.
Figure 2 shows the percent transmission of 300 - 500 nm light through phosphate buffered saline (PBS), plasma and platelets. Transmittance is almost 100% through PBS at this wavelength but very low for platelets and plasma.
Figure 3 shows an example of the effect of 100 mWcm⁻² 405 nm light on the CFU count of *Staphylococcus epidermidis, Staphylococcus aureus* and *Escherichia coli* suspended in human plasma at a population density of 10⁵ CFUml⁻¹.
Figure 4 is a plot of the effect of 405 nm light of differing irradiances on 10⁵ CFUml⁻¹ populations of *Staphylococcus aureus* suspended in human plasma.
Figure 5 is a plot of inactivation of 10¹, 10² and 10³ CFU/ml *S. aureus* in 3 ml platelet suspensions by exposure to 100 mW/cm² 405 nm light for 2-hr.
Figure 6 is a plot of inactivation of 10¹ CFU/ml *S. aureus* in 3 ml plasma using 14 mW/cm² 405 nm light.
Figure 7 is a plot of inactivation of *S. aureus* at a contamination level of 10³ CFUml⁻¹ in a 30 ml volume of human plasma held in a closed sample dish by exposure to ~8 mWcm⁻² 405 nm light.
Figure 8 is a plot of inactivation of *S. aureus* at a contamination level of 10³ CFUml⁻¹ in a 30 ml volume of human platelets held in a closed sample dish by exposure to ~8 mWcm⁻² 405 nm light.
Figure 9 is an optical analysis of transmission properties of the blood bag material and petri dish, highlighting 405 nm and UV-C light wavelengths for reference.
Figure 10 is a plot of 405 nm light inactivation of *S. aureus* suspended in human plasma covered by a layer of blood bag material.
Figure 11 is a plot of inactivation of *S. aureus* contamination in 300 ml transfusion bags of human plasma by exposure to 405 nm light using an irradiance of ~5 mWcm⁻².
Figure 12 is a plot of inactivation of ~10² CFU/ml *S. aureus* seeded into platelet bags (approx. 200 ml volume) using 3 mW/cm² 405 nm light.
Figure 13 shows the wavelength intensity spectrum for an exemplary light source centered around 405 nm discussed herein.
Figure 14 shows decreasing Feline Calici Virus (FCV) viral population in a minimal medium (MM) with increasing light doses.
Figure 15 shows decreasing FCV viral population in an organically-rich medium (ORM) with increasing light doses, with and without fetal bovine serum (FBS).
Figure 16 shows decreasing FCV viral population in both artificial saliva and in blood plasma with increasing light doses.
Figure 17 shows decreasing FCV viral population in artificial faeces with increasing light doses.
Figure 18 illustrates an optical analysis of the suspending media, demonstrating transmission of 405 nm light in various ORM, including blood plasma, showing fluorescence emission spectra of the various ORM when excited at 405 nm.

### DETAILED DESCRIPTION

### Explanation of Terms

The disclosed technology can be used to treat any of various stored blood products. As used herein, the term "blood products" includes whole blood, plasma, platelets, red blood cells, fluids comprising any of these substances, and/or fluids derived from any of these substances. The terms "blood" and "blood products" includes human, animal, and/or synthetic blood and blood products.

As used herein, the terms "pathogen" and "contaminant" and "contaminant microorganism" are used interchangeably and include any undesired bacteria, viruses, fungi, viroids, parasites, and/or other contaminant microorganisms present in a blood product. The term "bacteria" includes Gram positive types of bacteria, such as *Staphylococcus, Streptococcus, Bacillus, Clostridium, Enterococcus,* etc., species and also includes Gram negative types such as *Escherichia, Pseudomonas, Klebsiella, Acinetobacter,* etc., species. The term "fungi" includes single celled fungi such as yeasts and filamentous or spore forms such as mold fungi. The term "virus" includes any of the various types of viruses that can be present as contaminants in blood products.

As used herein, the term "inactivation" means that the subject microorganisms/pathogens are killed, damaged, and/or otherwise affected so as to inhibit or prevent their ability to replicate, infect a subject, and/or cause disease. For example, inactivation of a pathogen including viruses and other microorganisms stated in the above paragraph can include damaging the pathogen's genetic material that includes for example DNA, RNA, non-coding small RNAs (ncRNAs including microRNAs), and long non-coding RNAs (lnRNAs), thereby inhibiting its ability to reproduce, replicate and/or multiply. Methods disclosed herein can therefore be considered, in some cases, as bactericidal/fungicidal/viricidal or bacteriostatic/ fungistatic/virustatic and this may depend on the species/strain of bacteria or virus, wavelength of light, dose, and/or other factors.

Pathogen levels in blood products can be measured in terms of colony forming units, or "CFU", which is an estimate of the number of pathogens (e.g., cells, viruses, etc.) in a given volume of blood product that are viable and can multiply, reproduce, or replicate.

As used herein, the term "light" means electromagnetic radiation having specified wavelength properties. If not specifically limited to particular wavelength properties, "light" includes electromagnetic radiation having wavelengths from about 1 nm to about 1 mm, including ultraviolet (UV) light, visible light, and infrared (IR) light. The term "visible light" includes electromagnetic radiation having wavelengths from about 380 nm to about 800 nm. The term "violet-blue light" means electromagnetic radiation having wavelengths from about 380 nm to about 500 nm. The term "wavelength" means the length of one full wave or spatial period of the electromagnetic radiation, and can be defined as the inverse of the radiation's frequency.

As used herein, the terms "irradiance" and "intensity" are used interchangeably to mean the radiant flux received by a given surface per unit area, measured in watts per square meter (W/m²). Radiant flux is the radiant energy emitted/transmitted/received per unit time, and is measured in watts or joules per second. The term "radiation dose" or "light dose" means the product of the irradiance and the duration of exposure, and can be measured in joules per square meter (J/m²).

Light emitted or received can include a range of different wavelengths, and the light at each wavelength can have a different spectral irradiance. "Spectral irradiance" is the irradiance of a surface per unit wavelength. Often, light has a greatest spectral irradiance at a certain wavelength, and reduces in spectral irradiance at longer or shorter wavelengths, such as in a "bell curve" shaped pattern. The wavelength or wavelength range having the greatest spectral irradiance is referred to herein as the center wavelength or center wavelength range of the light, such that the light is referred to herein as being "centered on or around" the center wavelength or center wavelength range.

As used herein, the term "photosensitizing agent" means any chemical additive, not naturally present in blood, that is added to a blood product and that produces a chemical change in another molecule in a photochemical process. Exemplary photosensitizing agents include riboflavin, benzoporphyrin derivative-monoacid ring A (BPD-MA), phenothiazine derivative methylene blue and other dyes with photochemical properties including psoralens, such as S-59 (amotosalen-HCl) and S-303 or the ethylene imine PEN-110.

As used herein, the term "detrimental effect" means a substantial change in the normal physiological condition of a stored blood product (e.g., a substantial reduction in the ability of platelets to rapidly aggregate in response to TRAP introduction). Further, the phrase "without detrimental effect on the blood product" means without causing a substantial change in the normal physiological condition of the blood product.

### Exemplary Methods, Systems, and Devices

According to one aspect of the disclosed technology, there is provided methods and devices that utilize visible light at a wavelength and irradiation sufficient to inactivate pathogens (e.g., bacteria, viruses, fungi, viroids, prions, parasites) that are present in stored human and animal blood products, such as whole blood, plasma and platelets.

According to another particularly beneficial aspect, the disclosed technology utilizes visible light wavelengths (e.g., violet-blue light) without the use of an added photosensitising agent. Hence, a photosensitizing agent is not added to the blood products. Also since disclosed methods utilize visible light, this avoids the chemical and physical damage caused by the conventional methods of exposure to the higher energy photons of UV light.

According to another aspect of the disclosed technology, there is provided a method for inactivating bacteria, fungi, viruses, and/or other pathogens that can be present as contaminants in blood products, comprising exposure of the blood products to visible light having wavelengths in the range 380-500 nm. The light may have wavelengths in the range 400-450 nm. The light may have wavelengths in the range 380-420 nm. The light has a wavelength spectrum centered on or around 405 nm. In some embodiments, at least 50% or at least 90% of the light has a wavelength within any of the disclosed ranges. In some embodiments, at least 50% or at least 90% of the light has a wavelength within 10 nm of 405 nm.

According to another aspect of the disclosed technology there is provided a method and apparatus for the inactivation of pathogens in blood products while these are contained within sealed blood storage containers, such as blood transfusion bags. Microbicidal wavelengths of light are used that penetrate through the storage bag material and to sufficient depth within the blood product as to achieve effective pathogen inactivation over an exposure period.

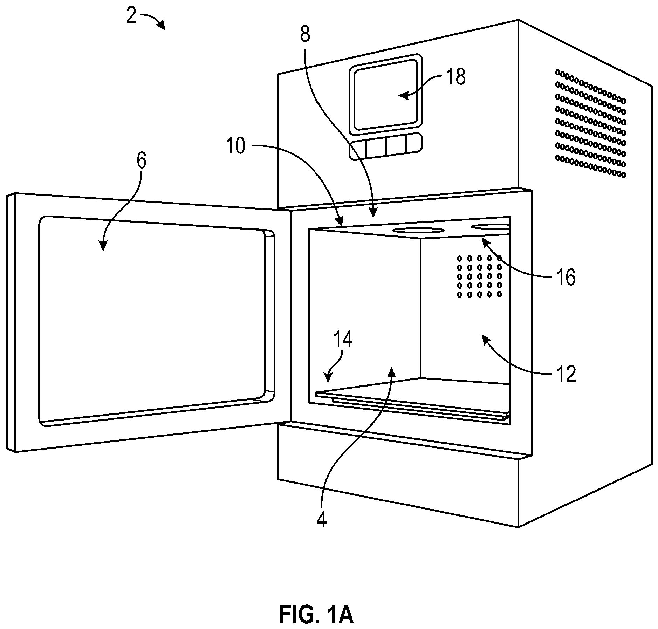

An exemplary treatment device 2 is illustrated in Figure 1A. The device 2 comprises an exposure chamber 4 wherein sealed blood product storage bags, or similar storage containers, are placed for treatment. The device 2 includes a door 6 that closes the treatment chamber and is configured to prevent some or all of the light of the selected emission wavelengths (e.g., 380 - 500 nm) from escaping from the treatment chamber. The door 6 can include a viewing window to observe the treatment process. The window can comprise a light filter (e.g., amber tinted) that allows certain visible wavelengths to pass through (e.g., 500 - 800 nm) and blocks shorter wavelengths. The door 6 can also include an automatic light turn-off system that turns off the lights when the door is opened.

The device 2 can utilize a lighting system 8 which can comprise one or more LED sources 10 and/or other types of light sources that emits light in a selected wavelength range and intensity sufficient to inactivate pathogens in stored blood products. The lighting system 8 can be configured to deliver light in any direction above, below and/or to the sides of the stored blood products in order to achieve focused or uniform illumination of the blood products as required. For example, light sources can be mounted on any surface of the chamber and/or on shelves, racks, trays, and/or other structures within the chamber. Blood storage bags can thereby be irradiated from two or more different directions, such as from the top and bottom, or two other opposing sides, at the same time.

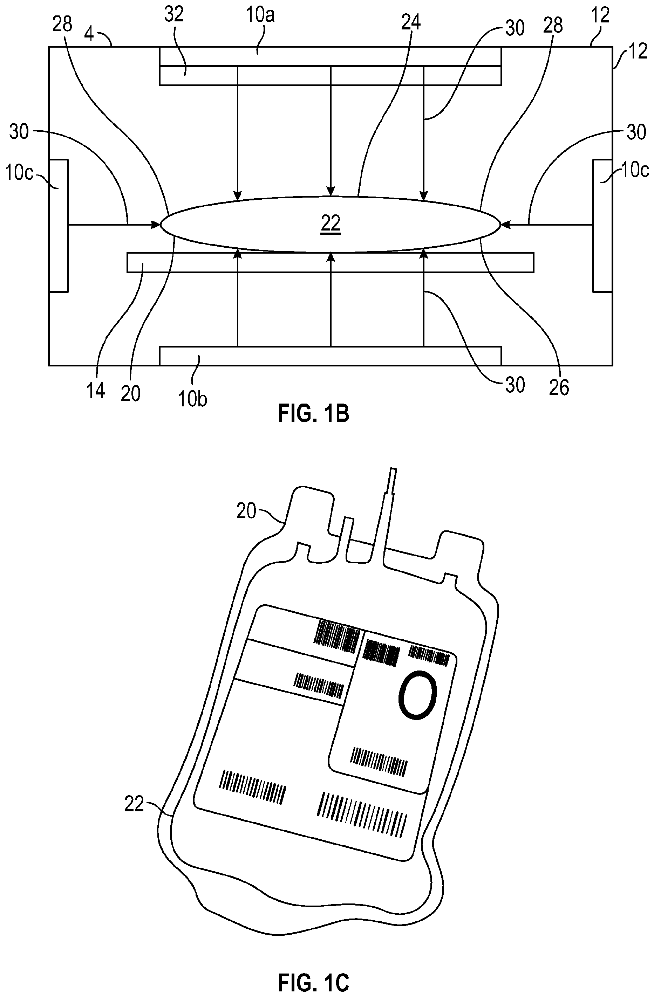

Figure 1B is a schematic view of an exemplary treatment 4 chamber of the device 2 with a storage container 20 containing a blood product 22 resting on a tray 14. Figure 1C shows an exemplary blood product storage container filled with a blood product. As shown in Figure 1B, the chamber 4 includes plural light sources, including an upper light source 10A, a lower light source 10B, and side light sources 10C. In a cuboid chamber, all four vertical walls can include light sources. Each light source emits light 30 that is directed to the storage container 20. The light 30 can reach and pass through some or all of the walls of the container 20, including the upper wall 24, lower wall 26, and side walls 28. In some embodiments, some of the light 30 can pass through the tray 14, such as to reach the lower wall 26. In some embodiment, the chamber 4 can also include reflective inner surfaces 12 to enhance irradiation of the container 20. The arrangement in Figure 1B is only one non-limiting example, and various other arrangements can be utilized in other embodiments.

The treatment chamber 4 can have various dimensions, even up to the size of an entire room, in which the lighting system 8 is installed at one or more locations around the treatment chamber, and the internal surfaces 12 of which can be lined with or made from highly reflective material. Exemplary reflective materials include various mirrored surfaces, polished stainless steel, polished aluminum, silver coated surfaces, various retroreflective material surfaces and surfaces that are optimal for reflecting light of wavelengths between 380 - 500nm and/or 400 - 420 nm and/or 405 nm.

According to another aspect of the disclosed technology, the treatment device can include a mechanically operated reciprocating tray or platform 14 on which stored blood products may be continuously agitated during exposure to the inactivating light. Agitation can help circulate the blood product inside the container for a more even irradiation and/or to reduce aggregation of platelets or other blood components. The reciprocating tray or platform 14 can be operated at different reciprocation rates (e.g., 10-100 rpm or 30-100 rpm) and for differing periods of time throughout the duration of the light treatment in order to provide sufficient mixing so that the opaque blood product receives sufficient light treatment within the illuminated zone under the semi-transparent bag material. As shown, the device 2 includes one tray 14, though in other embodiments the device can include two or more independent trays, such as trays that are stacked on top of each other and/or side-by-side. In some embodiments, the tray 14 or trays can be at least partially transparent or transmissive of the treatment light wavelengths such that blood product bags can be irradiated from below while resting on a tray. In embodiments with multiple trays, the light sources can be arranged and positioned around the chamber roof, walls, floor, and/or on the tray bottoms to provide the desired light exposure to the blood products on each tray. In various embodiments, the treatment chamber 4 can simultaneously contain two or more blood product containers in any relative orientation, such as lying side-by-side flat on one or more trays, one above the other on different trays, suspended from above or hanging, etc.

According to another aspect, the device 2 can comprise a temperature control system, ventilation system, and/or air circulation system 16 in order to accurately control the environment inside the chamber 4 throughout the treatment process.

According to another aspect of the disclosed technology, the lighting system 8 can include lenses or diffusers positioned adjacent to the light sources 10 in order to achieve directed or diffuse light outputs depending on the process requirements. For example, Figure 1B illustrates an exemplary lens and/or diffuser 32 coupled to the upper light source 10A. This can provide a more even light distribution and/or a more focused light exposure to a particular area.

According to yet another aspect of the disclosed technology, the lighting system 8 can deliver light at different intensities during the treatment period so that, for example, a high radiation dose may be delivered at the beginning of the process to achieve effective initial inactivation followed by a lower dose regime to maintain the decontamination effect. Predetermined intensity patterns and cycles can be used during a treatment. Other variations in dose delivery may also be used, such as depending upon the blood product type and nature of the pathogens present. For example, the intensity of the light emitted from the light sources can be sufficient such that irradiance levels at the surface of the blood product within the storage container can be from about 3 mWcm⁻² to about 80 mWcm⁻² in some embodiments. In some embodiments, the light reaching the surface of the blood product has an irradiance of 3 mWcm⁻² or less. In some embodiments the light reaching the surface of the blood product has an irradiance of 80 mWcm⁻² or greater. In some embodiments, the light emitted from the light sources can have any of the irradiance or intensity values provided herein, or greater values due to the reduced area of the light sources relative to the blood product. In some embodiments, the light reaching the external surface of the blood product storage container can have any of the irradiance or intensity values provided herein. In various embodiments, the light reaching at least some portion of the blood product within the storage container can have any of the irradiance or intensity values provided herein. The irradiance of the light can decrease as it passes through the storage container material, and thus the irradiance at the surface of the blood product can depend on the material and thickness of the storage container walls, as well as various other factors.

According to yet another aspect of the disclosed technology, the device 2 can have a lighting control system 18 that allows for the setting or programming of different irradiation intensities and durations throughout the blood product treatment period. The control system 18 can control the chamber air temperature and/or humidity, air circulation, tray agitation/vibration, light source selection, light source intensity, treatment duration, cycles, patterns, and/or automatic light source turn-off (e.g., when door is open or temperature is too high). The control system 18 can also include or be coupled to one or more sensors that measure desired properties and provide feedback to the control system 18. Exemplary sensors or sensor systems can include temperature sensors, light intensity or irradiance sensors, light wavelength or frequency sensors, tray weight sensors, and/or other sensors.

### Exemplary Investigations, Results, and Analysis

To investigate the effects of visible light, and particularly 405 nm light, on pathogens in stored blood products, the optical properties of blood, blood plasma, platelets, and other blood products were initially determined. To do this, spectrophotometric analysis was carried out over the wavelength range 220-700 nm using a Biomate 5 UV-Visible Spectrophotometer. The results shown in Figure 2 demonstrate that light transmission through plasma and platelets is below 5% over the 300-500 nm wavelength range, compared to >95% for a clear liquid such as phosphate buffered saline (PBS). Nevertheless, use of sufficiently high dose levels, multi-direction irradiation, agitation, and/or other techniques can be used to at least partly overcome transmissibility limitations of blood products.

The effectiveness of the visible light for inactivating pathogents in blood products was investigated by seeding bacteria into test liquids and then exposing them to visible light irradiation. Bacteria used in these tests were *Staphylococcus aureus, Staphylococcus epidermidis* and *Escherichia coli* and these were selected to represent significant contaminants associated with blood components.

The use of the bacteria described above was for illustrative purposes only. In addition, a wide range of other pathogens are susceptible to visible light, including 405 nm light. For example, Gram-positive and Gram-negative bacteria, yeasts, fungi, bacterial endospores and viruses can be inactivated by 405 nm light and/or other visible light, with up to 9-log reduction in populations demonstrated. Such pathogens can be successfully inactivated when suspended in blood products such as plasma and platelets when exposed to inactivating doses of 405 nm light and/or other visible light having bandwidths near 405 nm.

Disclosed devices and methods can be used for decontamination (e.g., inactivation of pathogens) of blood products, either within test containers or within blood transfusion storage bags. To test the effect of visible light on the inactivation of bacteria in selected blood products, defrosted fresh frozen human plasma and human platelets were seeded with known concentrations of bacterial contaminants. The light source used for exposure was an array of 405 nm LEDs powered by a direct current supply. For thermal management, the LED arrays were bonded to a heat sink and fan, thus ensuring that heating had no effect on the test samples exposed to the 405 nm light.

To demonstrate the principle, three different sample volumes (3 ml, 30 ml and approximately 300 ml (whole transfusion bags)) were used for exposure. For exposure of 3 ml samples, samples were held in 12-well microplates (without the lid), with the LED array positioned 3 cm above, providing an irradiance at the sample surfaces of ~100 mWcm⁻². 30 ml samples were exposed in 90 mm dishes (lid on), with the LED array positioned above to provide an irradiance of ~8 mWcm⁻². For exposure of platelet and plasma bags, LED arrays were held above the horizontally-positioned bags to give an irradiance of 3-5 mWcm⁻² at the center position of the bag. Experimental systems were held in a shaking incubator (72 rpm; 25°C) for continuous sample agitation to maintain exposure conditions. After light exposure, samples were plated onto growth medium, incubated, and enumerated to determine the number of surviving colony-forming units (CFU)/ml. The light dose (J cm⁻²) used to expose the blood samples was calculated as the product of the irradiance (W cm⁻²) multiplied by the exposure time (sec).

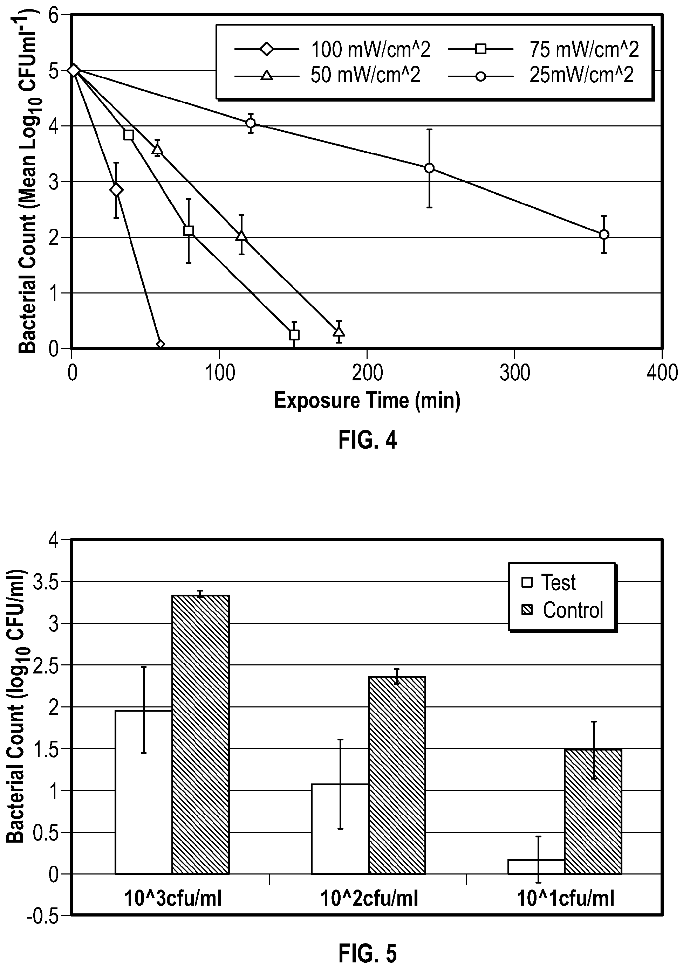

Figure 3 shows the effects of 100 mWcm⁻² 405 nm light for inactivation of *S. aureus, S. epidermidis* and *E. coli* in human plasma. Near complete inactivation of *S. aureus* was achieved after 45 min (270 Jcm⁻²). Exposure experiments repeated using lower irradiances of light (75, 50, 25 mWcm⁻²) also demonstrated significant population reductions, with use of higher irradiance resulting in increased inactivation rates (Figure 4). Similar inactivation kinetics were observed for *S. epidermidis.* Test data on *E. coli* also demonstrated that reduction of *E. coli* contamination can be achieved.

Tests carried out using 3 ml volumes of platelets seeded with different levels of bacterial contamination also demonstrate the ability of light of this wavelength to inactivate microbial contamination in platelets. Figure 5 shows the results from exposing 3 ml platelets seeded with *S. aureus* to 2-hr 100 mW/cm² 405nm light. Successful inactivation was achieved in all cases, with approximately 1.5 log₁₀ reductions being achieved after a 2-hr exposure to 100 mW/cm² light. Near-complete inactivation of 10¹ CFU/ml contamination was observed. Use of a longer exposure period results in complete inactivation of the 10² and 10³ CFU/ml contamination levels as was shown by use of a 4-hr exposure period, where near complete inactivation (<5 CFU remaining) of a 10³ CFU/ml population was achieved (data not shown).

Pathogens suspended in clear media, such as phosphate buffered saline (PBS), can be inactivated using lower doses of visible light. The need for higher doses in the case of inactivation in blood plasma and platelets can be accredited to the differing optical properties of these suspending media. The opacity, and consequent relatively low transmissibility of plasma and platelets (Figure 2), reduces photon penetration through the suspension, resulting in the need for greater doses, compared with suspension in clear, transparent liquids such as PBS. Despite this, these results demonstrate that significant inactivation of bacterial contaminants in human plasma can be achieved using visible light, such as light centered on or around 405 nm, with the higher the irradiance of light applied, the shorter the exposure time needed for successful inactivation. Ultraviolet light and other shorter-wavelength light, on the other hand, does not penetrate as well through opaque blood products, and consequently may not provide adequate inactivation of pathogens throughout a container under similar conditions.

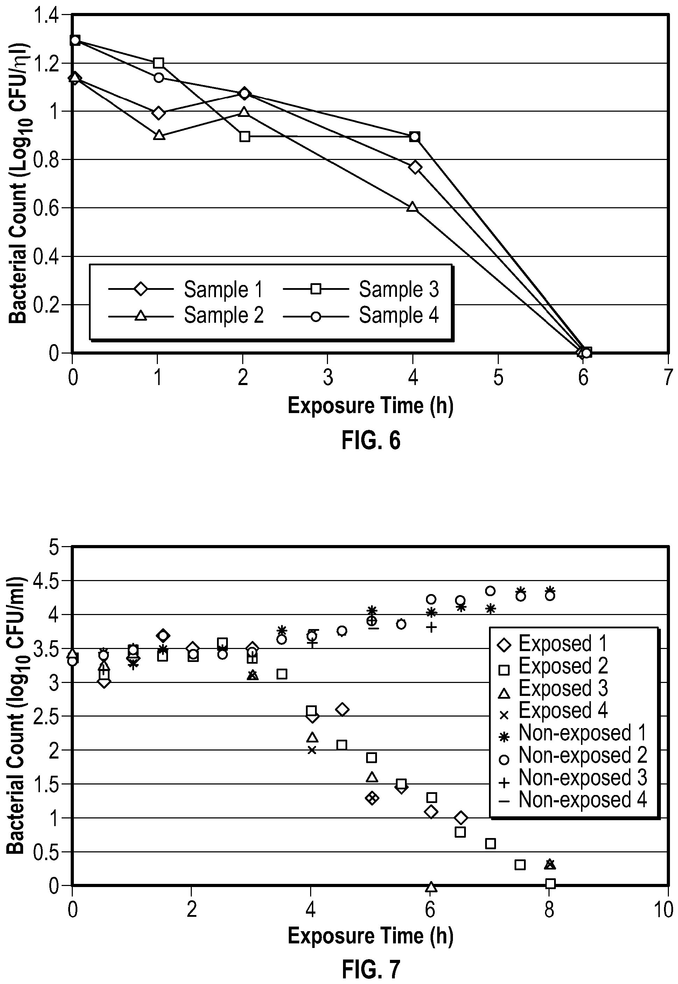

In addition to using high (e.g., 100 mWcm⁻²) irradiance levels at the blood product surface, experiments were carried out to establish that low density pathogen contamination (10¹ CFU/ml) can be inactivated when using a lower irradiance level, such as 14 mW/cm². Results, shown in Figure 6, demonstrate that complete inactivation of the bacterial contamination within plasma samples was achieved after a 6-hr exposure period. Control contamination levels remained unchanged.

To demonstrate that decontamination of larger volumes of plasma and platelets can be achieved (i) using lower irradiance visible light, and (ii) in sealed containers without the presence of an added photosensitizing agent, tests were conducted wherein 30 ml volumes of human plasma and platelets seeded with different levels of *S. aureus* were exposed, in a closed Petri dish, to an irradiance of ~8 mWcm⁻². An example of the results for plasma with a seeding density of 10³ CFUml⁻¹ is shown in Figure 7, and these results demonstrate that exposure of 8 hours at 8 mWcm⁻² (230 Jcm⁻² dose) induced near-complete inactivation. Similar results (not shown) were observed for inactivation of the 10² and 10¹ CFUml⁻¹ contamination levels, with near complete inactivation achieved with 187-230 Jcm⁻², and 201.6 - 230.4 Jcm⁻², respectively.

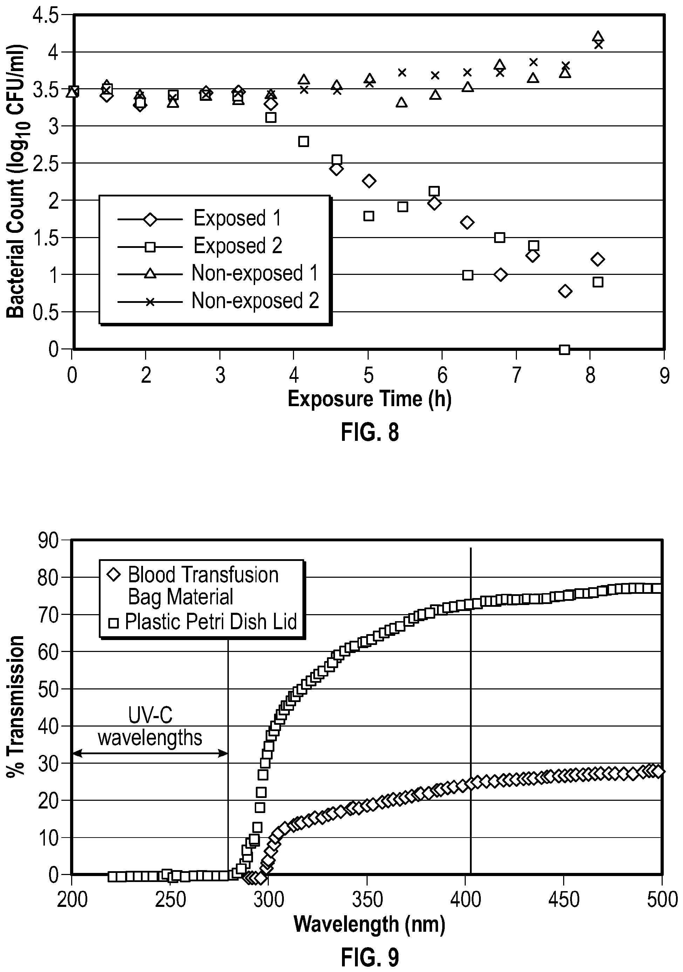

Figure 8 shows an example of the results demonstrating the inactivation of 10³ CFU/ml *S. aureus* in 30 ml platelet suspension in a closed dish using an irradiance of ~8 mW/cm². Inactivation became evident after 115 Jcm⁻² (4-hr), with near-complete inactivation achieved over the 8-hr exposure period (230 Jcm⁻²). Tests were also carried out (results not shown) demonstrating faster inactivation with a 10² CFU/ml contamination level with near complete inactivation achieved by 201 Jcm⁻². Successful inactivation of 10¹ CFU/ml contamination levels was achieved with the fastest rate, with complete/near complete inactivation achieved by 4-hr exposure (115 Jcm⁻²). Overall, these experiments successfully demonstrated that low irradiance light can be used over prolonged time periods to inactivate low-level contamination in 30 ml volumes of plasma and platelet suspensions, in sealed containers.

In order for the applied light to sufficiently affect the blood product, a sufficient amount of the applied light has to first pass through the storage container wall. For this reason, the transmissibility of blood product bag materials was measured and tested. Figure 9 shows the transmissability of a typical PVC blood product storage bag wall, as well as comparable transmissibility of clear plastic Petri dish wall, over different wavelengths. The measurements demonstrate that transmission of 405 nm light though the blood product bag material resulted in an approximate 20-30% loss in irradiance. However, light irradiance can be increased through the use of higher power light sources in order to compensate for this loss if needed. Alternatively or additionally, a storage container can be irradiated from opposite surfaces of the container. Overall, these results show that 405 nm light, and visible light in general, can transmit through typical blood product bag materials, thus permitting decontamination of at least the adjacent portions of the blood product contained in the storage bag. The transmissibility of visible light through storage container walls is also a significant advantage over UVC-light and other shorter-wavelength light, which is blocked nearly completely by the PVC bag material as shown in Figure 9.

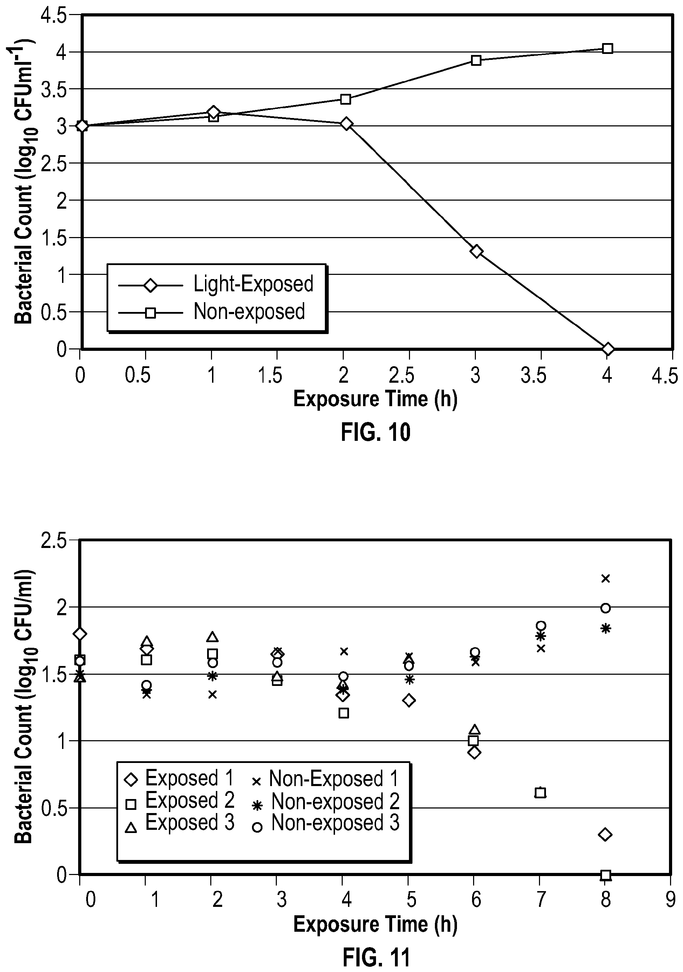

To further demonstrate the application of the disclosed technology, tests were carried out to establish that bacteria can be inactivated in human blood plasma when the applied light was transmitted through a layer of blood bag material. Tests were carried out by placing samples of the contaminated plasma into the wells of a 12-well microplate (without the lid), but with the wells covered with a layer of PVC blood-bag material, and the sample was exposed to 14 mWcm⁻² 405 nm light at the sample surface. Samples were treated in the rotary incubator (37°C; 72 rpm). Exposure of *S. aureus* suspended in human plasma to 405 nm light transmitted through the blood-bag material resulted in successful inactivation with complete inactivation achieved after a 4-hour exposure period as shown in Figure 10. The comparatively long exposure period in such cases can be attributed to the use of a low irradiance level of 14 mWcm⁻². Inactivation can be achieved more rapidly if a higher irradiance level is used for sample exposure.

To demonstrate the effectiveness of the disclosed technology for decontamination of plasma and platelets when these are contained within sealed blood bags, experiments were conducted to expose whole PVC transfusion bags, seeded with contamination, to 405 nm light. Inactivation of low density (10¹-10² CFU ml⁻¹) bacterial contaminants within PVC plasma transfusion bags was achieved using irradiances as low as 3 mWcm⁻² at the plasma surface. An example of inactivation using 5 mWcm⁻² at the plasma surface is shown in Figure 11. A downward trend in contamination was observed after exposure for 6h (108 Jcm⁻²). Complete/near complete inactivation was achieved after exposure for 8 hr (144 Jcm⁻²). This slightly reduced inactivation rate, compared to that found within the sample dishes, is due to the lower irradiance light being used for exposure: faster inactivation can be achieved using higher irradiance light, as shown in the 100 mWcm⁻² experiments.

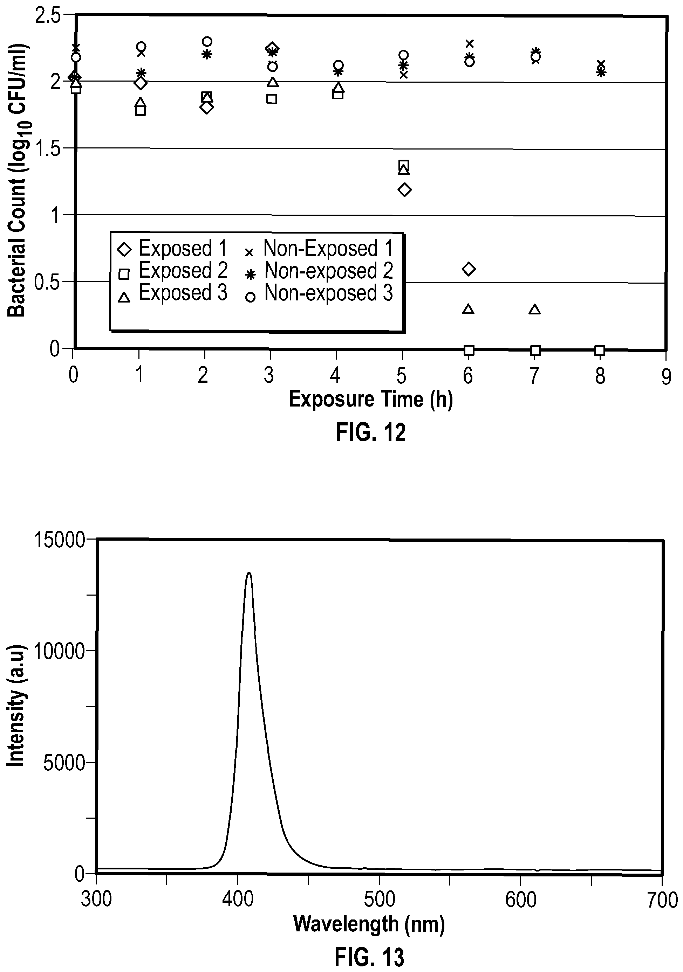

Figure 12 shows the successful results of the use of 5 mWcm⁻² 405 nm light for inactivation of contamination within platelet bags. Results demonstrate that bacterial inactivation becomes evident after 5h exposure (90 Jcm⁻²), with complete/near complete inactivation being typically achieved by 6 hours (108 Jcm⁻²). Control bacterial populations in the non-exposed samples demonstrated that the bacterial contamination either remains unchanged or increases slightly over the course of the 8-hour period, confirming that the inactivation effect was the result of the light exposure. The inactivation kinetics achieved when the bags were exposed to four different irradiance levels (3, 5, 7, 10 mWcm⁻² - data not shown) appeared to follow similar trends, indicating that differences in irradiances in the region of 3-10 mW/cm² are not likely to have a major effect on the inactivation rates. This may be aided by the agitation of the bags during exposure, which facilitates circulation of the blood product and thus good illumination of the whole volume of blood product.

The preceding investigations and results demonstrate that the disclosed technology effectively inactivates pathogens contained within blood products, such as blood plasma and platelets. Furthermore the disclosed technology establishes that visible light and particularly 405 nm light can be used for inactivation of pathogens in plasma and platelets while these blood products are contained within transfusion bags. The fact that light of 405 nm wavelength can penetrate the transfusion bag material enables the blood products to be decontaminated (i.e., inactivation of pathogens) while sealed in the bags, thereby significantly reducing the risk of any post treatment contamination that can occur with other procedures.

When the disclosed technology is used as a platelet decontamination technology, it can be important that the visible light treatment has no adverse effects on the platelet cells, and that they are suitable for transfusion post treatment. Testing and establishing such features of the disclosed technology included the establishment of information about the physiological effects of visible light on platelet cells. To this end, the standard tests that are performed on platelets in blood bank storage conditions, such as platelet count, pH of the platelet solution, and platelet aggregation in response to platelet agonists [e.g. Thrombin Receptor Agonist Peptide (TRAP)], were evaluated following a selected time (in hours) of 405 nm light exposure of platelets in the bags (see Tables 1a, 1b and 1c below). Rapid aggregation associated with TRAP induction is a positive indicator that platelets are in normal physiological conditions during storage. Thus, the test to control ratio of 1 (i.e., 100%) or close to 1 (i.e., close to 100%) is a good indicator of the quality of platelets in any given context.

Analysis was performed using platelets processed from a donor split into two equal halves and each half was stored in a storage bag. At least two donor samples were used for each experiment (n=2), where feasible. For each 405 nm light exposure treatment, one platelet bag was used as control and the other as test sample. Platelet bags were exposed to three different treatment conditions: [1] At 10 mW/cm² 405nm light for up to 6 hrs; [2] At 40 mW/cm² 405nm light for up to 6 hrs; and [3] At 80 mW/cm² 405nm light for up to 6 hrs. The non-exposed control samples (in a light-safe box) and the test samples were held under rotary conditions during exposure (~25°C and 72 rpm) in the same incubation chamber. Samples were collected and analyzed at 2 h intervals from both light-exposed and control samples.

Tables 1a, 1b, and 1c below show platelet counts, pH measurements and TRAP-induced platelet aggregation of non-exposed platelets and platelet samples exposed to 405 nm light at 10 mW/cm² (Table 1a) 40 mW/cm² (Table 1b) and 80 mW/cm² (Table 1c) for up to 6 hrs while in transfusion bags. Note that "Control" and "Test" are two individual bags originated from the same donor and the same donation (split sample).

**Table 1a (405 nm light 10 mW/cm²)**

| Exposure (h) | Platelet count (K/µl) | | pH | | TRAP-induced aggregation shown as test to control ratio (%) |
|---|---|---|---|---|---|
| | Control | Test | Control | Test | |
| 0 | 1295 | 1301 | 7.2 | 7.1 | 100* |
| 2 | 1295 | 1309 | 7.2 | 7.1 | 100* |
| 4 | 1288 | 1304 | 7.1 | 7.1 | 100 |
| 6 | 1277 | 1279 | 7.2 | 7.1 | 100 |

**Table 1b (405 nm light 40 mW/cm²)**

| Exposure (h) | Platelet count (K/µl) | | pH | | TRAP-induced aggregation shown as test to control ratio (%) |
|---|---|---|---|---|---|
| | Control | Test | Control | Test | |
| 0 | 1936 | 1930 | 7.1 | 7.1 | 100* |
| 2 | 1932 | 1920 | 7.0 | 6.9 | 106 |
| 4 | 1925 | 1900 | 7.1 | 6.9 | 118 |
| 6 | 1916 | 1924 | 7.1 | 6.9 | 45 |

**Table 1c (405 nm light 80 mW/cm²)**

| Exposure (h) | Platelet count (K/µl) | | pH | | TRAP-induced aggregation shown as test to control ratio (%) |
|---|---|---|---|---|---|
| | Control | Test | Control | Test | |
| 0 | 1634 | 1596 | 7.0 | 7.0 | 100* |
| 2 | 1490 | 1520 | 7.1 | 7.0 | 96 |
| 4 | 1481 | 1492 | 7.1 | 6.7 | 46 |
| 6 | 1476 | 1474 | 7.2 | 6.5 | 0 |

Overall, the results of the analytical studies carried out (platelet count, pH and TRAP-induced aggregation) on platelets exposed to 405 nm light show that there are no major physiological changes in the platelet cells when exposed in containers or transfusion bags to 405 nm irradiances at 10 mW/cm² for up to 6 hours (Table 1a), or 40 mW/cm² for 4 hours (Table 1b), or 80 mW/cm² for up to 2 hours (Table 1c). However, change in pH and TRAP-induced aggregation was prominent when 405 nm irradiance at 80 mW/cm² was extended to 6 hours. These irradiance levels and treatment times have been shown to successfully inactivate the low levels of microbial contamination that can occur in whole blood platelet and plasma transfusion bags due to the light transmission through the sealed bag material and into the stored product. These exposure conditions can be compatible with the typical storage conditions of the platelets, so that one application for the technology is to utilize a combined storage and treatment regime involving exposing the platelets to light having wavelength spectrum centered on or around 405 nm while agitating the platelets during storage until they are used for transfusion.

Some embodiments of the disclosed technology utilize high irradiance treatment (e.g., up to 80 mW/cm²) for exposure of whole platelet/plasma bags in order to achieve shorter exposure periods (e.g. minutes) for the effective inactivation of pathogens in the blood products without having detrimental effects on the blood products. Results from the analysis of high irradiance light-exposed platelets showed that there were no significant detrimental changes apparent in the platelet suspensions. Accordingly, some embodiments of the disclosed technology utilize high-irradiance exposure of whole bags to enable platelet/plasma bags to be treated quickly (e.g., 30-60 mins) immediately after collection from the donor before being stored awaiting transfusion.

The disclosed technology can be used to treat blood products stored in many different types of storage containers comprising various materials, so long as the container provides at least some transmissivity to the applied light. Exemplary container/bag materials suitable for use with the disclosed technology include PVC (polyvinyl chloride), EVA (ethylene-vinyl acetate), PE (polyethylene), PP (polypropylene), PS (polystyrene), combinations of such materials, variations of such materials, and/or other at least partially light-transmissive materials.

### Viral Inactivation

Figures 13-18 illustrate the efficacy of the disclosed technology for viral inactivation in blood products and other media. The following describes testing that was performed to validate the efficacy of the disclosed technology involving 405 nm light (see spectrum in Figure 13) in inactivating feline calicivirus (FCV), which serves as a model for Norovirus (NoV), which is one of the most common causes of epidemic acute gastroenteritis and can be transmitted via food and water, person-to-person contact or environmental surfaces contacts. Environmental stability and resistance to disinfection further aids transmission of NoV, with viral particles detected on surfaces up to 42 days after contamination. If environmental decontamination is deficient this can lead to ward closures which has substantial operational and financial implications for health boards. NoV outbreaks in the healthcare setting and other densely populated areas such as nursing homes and schools and restaurants have driven the need for new decontamination systems. However, the antiviral efficacy of 405 nm light on medically important human and animal viruses was lacking and required investigation.

The study described here was designed to illustrate the interaction of narrowband 405 nm light with FCV as a model to study the antiviral effects of this light on NoVs. FCV was selected as a NoV surrogate, as there is currently no standardized cell-culture system for NoV. Our data demonstrates the influence of the suspending media, including biologically-relevant fluids, on viral susceptibility. As such, this study provides evidence of the antiviral efficacy and discusses the potential mechanism of 405 nm light viral inactivation.

Feline embryonic cells, strain FEA (Jarrett, Laird & Hay 1973) were cultured in Dulbecco's Modified Eagle's Medium (DMEM) supplemented with 10% fetal bovine serum (FBS), 2 mM L-glutamine, 1 mM sodium pyruvate and 240 U mL⁻¹ penicillin streptomycin (Gibco, Life Technologies, UK), to form 10% FBS-DMEM. Cells were maintained at 37°C in 5% CO₂.

To prepare a virus pool of the FCV vaccine strain F9, virus inoculum (School of Veterinary Medicine, University of Glasgow) was added to FEA monolayers in 850 cm² cell culture roller flasks (Corning, USA). After 90-min incubation of the inoculated cells on a rotating roller stand at 37°C in 5% CO₂, fresh culture medium was added and flasks incubated for 24 hours. This resulted in virus-induced destruction of nearly 90% of the cell monolayer.

The tissue culture supernatant, and medium from a single wash step, was collected from each roller bottle and subjected to two freeze-thaw cycles before clarification by centrifugation at 3300 × g for 10 minutes. The virus-containing supernatant was then stored at -80°C until required. The infectious titre of FCV was approximately 2 × 10⁷ plaque forming units per millilitre (PFU mL⁻¹), determined by standard plaque assay techniques (Ormerod & Jarret 1978).

The light source used was a 405 nm light emitting diode (LED) array (ENFIS PhotonStar Innovate UNO 24; PhotonStar Technologies, UK) powered by a 40 V Phillips Xitanium LED Driver (Phillips, Netherlands). The array had a peak wavelength around 405 nm and a bandwidth of approximately 19 nm (Figure 13) but will, for convenience, be referred throughout the following text as 405 nm light. The array was attached to a heatsink and cooling fan, to minimize heat transfer to test samples, so that no significant heating of the sample occurred. The light source was held on a PVC stand at a distance of 4 cm from the microbial samples, giving an irradiance of 155.8 mW cm⁻² at the sample surface (measured using a radiant power meter and photodiode detector (LOT Oriel, USA)).

FCV stock virus was defrosted at room temperature and diluted to 2 × 10⁵ PFU mL⁻¹ in Dulbecco's phosphate buffered saline, supplemented with calcium and magnesium (DPBS; Hyclone, Thermo Fischer Scientific, UK). This was used as a 'minimal medium' (MM). 1.5 mL volumes of viral suspension were transferred into the central 4 wells of a 24-well plate (Techno Plastic Products, Switzerland) and the plate positioned on a raised stand, with the sample wells 4 cm directly below the light source and the plate lid kept on to prevent evaporation. Test samples were exposed to increasing doses of 405 nm light at room temperature, with the dose calculated as the product of irradiance (mW cm⁻²) × exposure time (s). Control samples were set up under identical environmental conditions but without 405 nm light illumination. Post-exposure, FCV samples were immediately removed from the well and serially diluted in MM for enumeration by plaque assay.

Exposures were repeated with FCV suspended in 'organically-rich media' (ORM): DMEM, 10% FBS-DMEM, artificial saliva, artificial faeces and blood plasma. The artificial saliva was a modified version of that used by Margomenou *et al.* (2000) [5.2 g NaHCO₃, 0.88 g NaCl, 1.36 g K₂HPO₄, 0.48 g KCl, 2000 units α-amylase and 2 g pig gastric mucin (Sigma Aldrich, UK) in 1 L sterile water], and was adjusted to pH 7-7.5 to emulate the variability of pH in human saliva, and also to ensure no FCV inactivation occurred (Duizer *et al.* 2004b; Edgar *et al.* 2004). The artificial faeces was a modified version of that by Colón *et al.* (2015) [30g inactivated yeast (Marigold, UK), 7 g physillum (Buy Whole Foods Online, UK), 11g miso paste (Yutaka, UK), 8 g cellulose, 1.6 g NaCl, 0.8 g CaCl, 1.6 g KCl (Sigma Aldrich, UK) in 920 mL sterile water], and was also adjusted to pH 7. The modifications to the artificial saliva and faeces formulations were to ensure compatibility with the FEA cells. Fresh frozen human blood plasma was obtained from the Scottish National Blood Transfusion Service (SNBTS, UK), and defrosted before use. FCV was also exposed whilst suspended in MM supplemented with riboflavin, with and without tyrosine, tryptophan, pyridoxine and folic acid (used at the same concentrations as found in DMEM: 0.4, 104, 16, 4 and 4 mg L⁻¹ respectively).

Prior to experiments, 6-well cell culture plates (Thermo Fischer Scientific) were seeded with 7.5 × 10⁵ FEA cells per well. 3 mL of the cell suspension in growth medium was pipetted into each well, and incubated at 37°C in 5% CO₂ for 20-h, resulting in confluent monolayers.

Post-exposure of FCV, the growth medium was aspirated from the FEA cells and replaced with 1 mL FCV sample. Plates were co-incubated at 37°C in a humidified 5% CO₂ incubator for 90-min, with the plates gently rocked every 15-min to ensure even distribution of the inoculum over each monolayer.

After the viral incubation period, the inoculum was aspirated and the well washed with medium (10% FBS-DMEM or DPBS) before adding 4 mL overlay mixture consisting of 2× supplemented DMEM 1:1 with 2× agarose. 2× supplemented DMEM was prepared using 20 mL from a filter sterilized stock of 10× DMEM, adding the same supplements as detailed earlier, plus 9.86 mL sodium bicarbonate solution (Gibco), and was made up to 100 mL with sterile water. 2× agarose was prepared by dissolving 2 g agarose (Sigma Aldrich) in 100 mL deionized distilled water and sterilized by autoclaving. The overlay was left to set before the plates were incubated for 44-48 hours at 37°C in 5% CO₂.

Post-incubation, the monolayers were fixed and stained overnight with 0.5% crystal violet in 10% buffered neutral formalin. The agarose plugs and stain were then removed, the plates left to dry, plaques counted, and the virus infectivity titre expressed as PFU mL⁻¹.

The presence of porphyrins, or other components with the ability to absorb 405 nm light and emit fluorescence, within the suspending media was determined by fluorescence spectrophotometry. Media were freshly prepared and fluorescence measurements were carried out using a RF-5301 PC spectrofluorophotometer (Shimadzu, USA). Excitation was carried out at 405 nm and emission spectra recorded between 425-700 nm.

Data points represent mean results ± standard deviation (SD), taken from triplicate independent experiments (n=3). The antiviral activity of 405 nm light was determined by calculating the reduction in the level of infectivity from the difference between Log₁₀ values for exposed and control samples. Significant differences were calculated using one-way ANOVA (Minitab 16 Statistical Software) with results found to be significant when P<0.05.

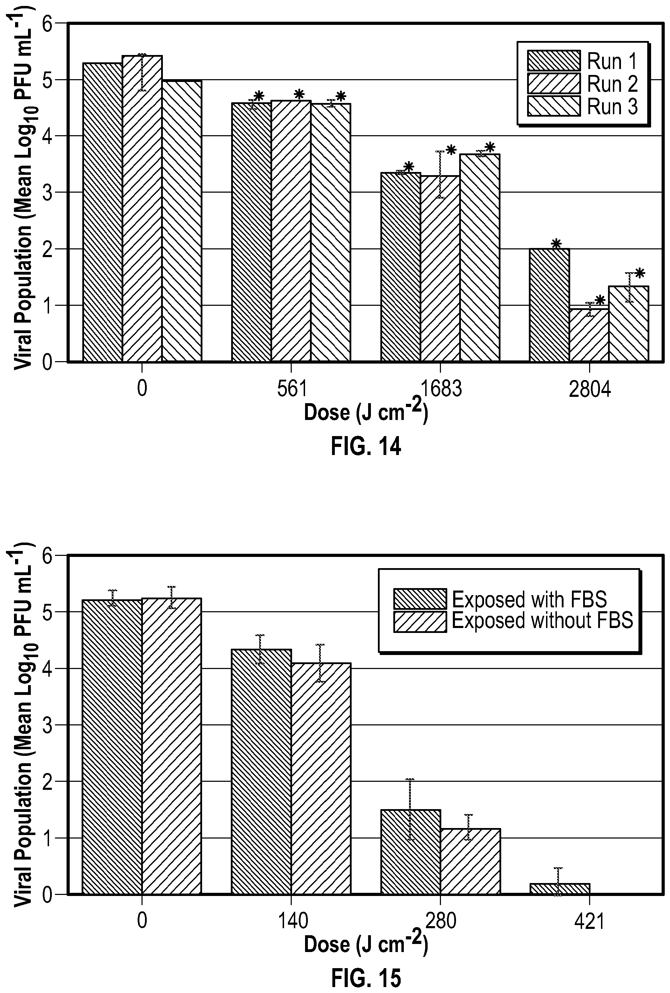

FCV was suspended in MM and ORM and exposed to increasing doses of 405 nm light at an irradiance of 155.8 mW cm⁻². Results (Figure 14) show that when suspended in MM, significant FCV inactivation was achieved after exposure to 561 J cm⁻² (*P*=0.043), and relatively linear inactivation kinetics were observed, with a dose of 2.8 kJ cm⁻² required for a 3.9 Log₁₀ inactivation. The non-exposed control samples showed no significant change over the course of the experiment *(P>0.05).*

Antiviral efficacy was found to differ significantly when suspended in ORM. When exposed in 10% FBS-DMEM, a significantly lower dose was required for viral inactivation (Figure 15), with a 4.8 Log₁₀ reduction achieved after a dose of 421 J cm⁻². As the presence of FBS in DMEM is thought to reduce the level of oxidation upon exposure to normal laboratory lighting (Grzelak *et al.* 2001), the exposure was repeated with FCV suspended in DMEM without FBS to observe any differences in inactivation kinetics. Although slightly less inactivation was observed with each applied dose, results (Figure 15) demonstrate no significant differences in the inactivation kinetics of FCV when the virus is exposed in DMEM in the presence or absence of 10% FBS (*P*>0.05). Control samples showed no significant decrease (*P*>0.05).

Furthermore, components of DMEM have been shown to be photosensitive to light (Grzelak *et al.* 2001), therefore exposures were repeated with riboflavin added to MM with and without tyrosine, tryptophan, pyridoxine and folic acid in the same concentrations found in DMEM (see Table 2 below). Results demonstrated that exposure of FCV suspended in MM with riboflavin only resulted a 1.3 Log₁₀ reduction after 421 J cm⁻¹, however when all components were present enhanced inactivation occurred and a 5.1 Log₁₀ inactivation was achieved.

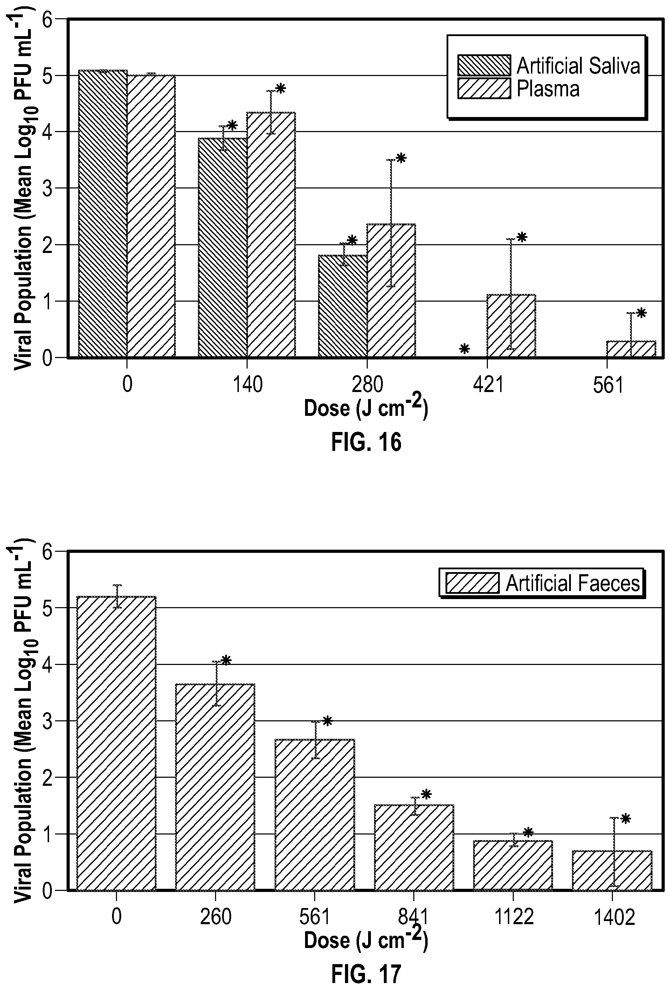

Artificial saliva, artificial faeces, and blood plasma were selected as ORM which are biologically-relevant in terms of media in which viral particles may be found in the environment, with Norovirus regularly identified in faeces. Exposure of FCV when suspended in artificial saliva yielded results similar to those in DMEM, with a 5.1 Log₁₀ reduction of infectivity achieved after a dose of 421 J cm⁻² (Figure 16). (In this case, inactivation was measured to a sensitivity of ten PFU mL⁻¹, as the artificial saliva in the undiluted samples adversely reacted with the FEA cells causing them to dislodge from the plate). The dose required for inactivation when suspended in blood plasma was slightly greater than when in artificial saliva, with 561 J cm⁻² required for 4.8 log₁₀ inactivation of FCV (Figure 16). FCV inactivation in artificial faeces required greater doses, with 4.5 log₁₀ inactivation achieved after 1.4 kJ cm⁻² (Figure 17). Control samples in artificial saliva, plasma and artificial faeces showed no significant change (*P*=0.618, 0.101, 0.747, respectively).

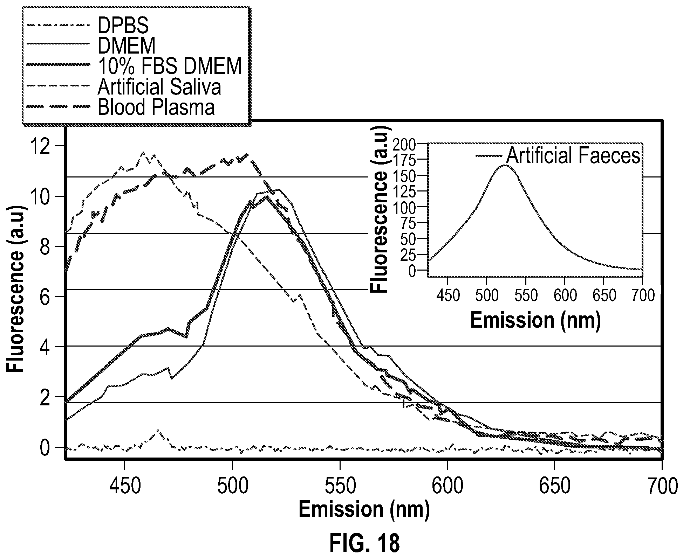

The fluorescence emission spectra (Figure 18) of MM (DPBS) and ORM (DMEM, 10% FBS-DMEM, artificial saliva, plasma and artificial faeces) when excited at 405 nm, shows emission peaks for DMEM, 10% FBS-DMEM, plasma and artificial faeces observed between 510-520 nm and for artificial saliva at 460 nm.

**Table 2: Comparison of the inactivation of feline calicivirus (FCV) when suspended in minimal media supplemented with riboflavin alone or alongside tyrosine, tryptophan, pyridoxine and folic acid, upon exposure to 405 nm light at an irradiance of 155 mW cm⁻². Data points represent the mean count (n=3) ± SD. Asterisks indicate light-exposed samples that were significantly different to the non-exposed final control samples (P≤0.05).**

| **Photosensitive Components** | **Starting Population (±St Dev)** | **Exposed Viral Population (±St Dev)** | **Non-Exposed Control Population (±St Dev)** | **Log₁₀ Reduction** |
|---|---|---|---|---|
| | 5.01 ± 0.02 | 3.77 ± 0.61 | 5.05 ± 0.06 | 1.28* |
| **Riboflavin** | | | | |
| **Riboflavin, Tyrosine, Tryptophan, Pyridoxine Folic Acid** | 5.15 ± 0.03 | 0.00 ± 0.00 | 5.12 ± 0.07 | 5.12* |

Although there has been a recent move towards using murine norovirus and tulane virus as NoV surrogates (Cromeans *et al.* 2014; Kniel 2014), FCV was chosen as it has physiochemical and genomic similarities to NoV, and is a well-established surrogate with a standardized cell-culture protocol (Doultree *et al.* 1999; Bidawid *et al.* 2003; Duizer *et al.* 2004a, b; Chander *et al.* 2012). Similarly, studies investigating the virucidal effects of UV-light, ozone, hydrogen peroxide vapour and cold atmospheric gas plasma technologies have also used FCV as a NoV surrogate (Nuswalen *et al.* 2002; Hudson *et al.* 2007; Bentley *et al.* 2012; Aboubaktar *et al.* 2015; Holmdahl *et al.* 2016).

The virucidal efficacy of 405 nm light was determined using FCV suspended in both minimal medium (MM) and organically-rich media (ORM). Exposure in MM would provide a better indication of the interaction of 405 nm light and the virus alone, whilst suspension in ORM, which is likely to contain photosensitive components, would assess how viral susceptibility can potentially be influenced by the surrounding media.

Successful FCV inactivation was achieved when suspended in MM, although the dose required was great, with 2.8 kJ cm⁻² achieving a 3.9 Log₁₀ reduction (Figure 14). In the case of bacteria and fungi in MM, doses in the range of 18-576 J cm⁻² are typically required for 5 Log₁₀ inactivations (Maclean *et al.* 2009; Murdoch *et al.* 2012; Murdoch *et al.* 2013). The increased susceptibility of bacteria and fungi compared to viruses is accredited to the presence of endogenous photosensitive porphyrins within these cells (Hamblin *et al.* 2004; Maclean *et al.* 2008; Murdoch *et al.* 2013). Low sensitivity of FCV in MM occurred due to the absence of porphyrins in the viral structure, coupled with the fact that MM does not contain any photosensitive substances which absorb light at 405 nm (Figure 18), suggesting that viral inactivation, in this case, is due to a differing mechanism.

Inactivation in MM may be associated with the LED emission spectrum extending slightly into the UVA region (Figure 13), meaning the virus is exposed to very low-level UVA photons (~390 nm). Over an extended period, this could cause oxidative damage to proteins (Girard *et al.* 2011) for example to the viral capsid, and therefore contribute to the observed inactivation. Another possibility is that the small amount of 420-430 nm light emitted from the source may contribute to viral inactivation. Antiviral effects of 420-430 nm have been demonstrated against murine leukemia virus, with long exposures thought to cause photo-damage to the virion-associated reverse transcription complex (Richardson & Porter 2005). Although the virus differs in structure to FCV, these findings suggest that prolonged exposure to wavelengths at the tail ends of the 405 nm LED emission spectrum such as 390 and 420 nm, as well as 405 nm, may affect the viruses' ability to infect and replicate in host cells, and have a role in the inactivation of FCV by the LEDs used in this study.

To investigate whether exposure in ORM had any effect on viral susceptibility, FCV was first suspended in DMEM with and without 10% FBS (thought to aid protection against ROS (Grzelak *et al.* 2001)). Results (Figure 15) demonstrated near complete reduction in infectivity of a 10⁵ PFU mL⁻¹ population after a dose of 421 J cm⁻². As can be seen in Figure 3 slightly greater inactivation occurred when FCV was suspended in DMEM without the FBS serum additive, however, no significant difference was seen between the inactivation kinetics. As the inactivation dose of 421 J cm⁻² is 85% less than that required for a similar level of inactivation in MM, it is likely that components of the ORM are influencing FCV inactivation. A study investigating the susceptibility of bacteriophage ϕC31 demonstrated similar results to those of the current study: little inactivation was observed when exposed in a simple salt solution, however susceptibility was significantly enhanced when suspended in a nutrient-rich medium, with a 5.4 Log₁₀ reduction of ϕC31 achieved after exposure to 510 J cm⁻². This was hypothesized to be due to the complex protein and amino acid rich composition of the nutrient-rich medium, suggesting some components could be photosensitive and when exposed to 405 nm light in the presence of oxygen would produce ROS, damaging the bacteriophage. This same phenomenon is likely to account for the enhanced inactivation of FCV when in DMEM and 10% FBS-DMEM, as these contain a complex mixture of amino acids, vitamins and sugar, which have the potential to absorb 405 nm light (Figure 18) and act as photosensitizers.

The photosensitization of components of DMEM has also been demonstrated upon exposure to light, with riboflavin shown to produce ROS which is further enhanced by tryptophan, tyrosine, pyridoxine and folic acid (Grzelak *et al.* 2001). Furthermore, blue light wavelengths are thought to be the most efficient for the photo-decomposition of riboflavin and generation of ROS (Cheng *et al.* 2015). To investigate this, riboflavin was added to MM with and without tyrosine, tryptophan, pyridoxine and folic acid in the same concentrations found in DMEM (Table 2 above). Results support this with only 1.3 Log₁₀ reduction when only riboflavin was present, however when all components were present enhanced inactivation of FCV was achieved with complete inactivation of a 10⁵ PFU mL⁻¹ population.

It is important to consider how light-induced inactivation would be influenced when viral particles were suspended in more biologically-relevant, naturally occurring matrices such as body fluids or secretions. As artificial saliva and artificial faeces can be prepared, these were used alongside human plasma, as model human secretions in which many viruses can be transmitted (Aitken & Jeffries, 2001).

Results (Figures 16 and 17) demonstrated that, similarly to inactivation in ORM (DMEM and 10% FBS-DMEM), viral susceptibility was significantly increased when suspended in these biologically-relevant fluids. Of the three model fluids used, sensitivity was highest when suspended in saliva, with a 5.1 Log₁₀ reduction of FCV infectivity achieved after a dose of 421 J cm⁻² - the same as that observed when in ORM. Susceptibility was slightly reduced when suspended in blood plasma (4.8 Log₁₀ inactivation with 561 J cm⁻²), and further reduced when in artificial faeces, with more than three times the dose required to achieve a 4.5 Log₁₀ reduction. The reduced levels of 405 nm light transmission through the plasma and artificial faeces will contribute to these slower inactivation rates. Overall, the susceptibility of FCV to 405 nm light when suspended in artificial faeces, artificial saliva, blood plasma and other organically-rich media was significantly increased when compared to susceptibility in minimal media, with 50-85% less dose required for similar levels of viral inactivation. Inactivation when suspended in these ORM is likely due to the proteins contained within the media, for example, the mucin in the artificial saliva, proteins within the plasma, and inactivated yeast within the artificial faeces, which may all be predisposed to photosensitization (demonstrated by the fluorescence peaks around 460 nm and 510-520 nm in Figure 18). These results indicate that NoV susceptibility to 405 nm light can be enhanced when in ORM, or host secretions in which they are released such as faeces, blood and vomit. Although the consistency and transparency/opacity may differ to those used in this study, these fluids are likely to be rich in molecules which could be sensitive to 405 nm light, thereby aiding in NoV inactivation.

The disclosed technology can also provide for beneficial application of 405 nm light for the decontamination of air, surfaces and equipment in healthcare settings, as well as in other indoor locations, where transmission of viral pathogens is a significant occurrence.

For purposes of this description, certain aspects, advantages, and novel features of the embodiments of this disclosure are described herein. The disclosed methods, apparatuses, and systems should not be construed as limiting in any way. Instead, the present disclosure is directed toward all novel and nonobvious features and aspects of the various disclosed embodiments, alone and in various combinations and sub-combinations with one another. The methods, apparatuses, and systems are not limited to any specific aspect or feature or combination thereof, nor do the disclosed embodiments require that any one or more specific advantages be present or problems be solved.

Integers, characteristics, materials, and other features described in conjunction with a particular aspect, embodiment, or example of the disclosed technology are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments.

Although the operations of some of the disclosed methods are described in a particular, sequential order for convenient presentation, it should be understood that this manner of description encompasses rearrangement, unless a particular ordering is required by specific language. For example, operations described sequentially may in some cases be rearranged or performed concurrently. Moreover, for the sake of simplicity, the attached figures may not show the various ways in which the disclosed methods can be used in conjunction with other methods.

As used herein, the terms "a", "an", and "at least one" encompass one or more of the specified element. That is, if two of a particular element are present, one of these elements is also present and thus "an" element is present. The terms "a plurality of" and "plural" mean two or more of the specified element. As used herein, the term "and/or" used between the last two of a list of elements means any one or more of the listed elements. For example, the phrase "A, B, and/or C" means "A", "B,", "C", "A and B", "A and C", "B and C", or "A, B, and C." As used herein, the term "coupled" generally means physically, magnetically, electrically, wirelessly, or otherwise coupled or linked together and does not exclude the presence of intermediate elements between the coupled items absent specific contrary language.

In view of the many possible embodiments to which the principles of the disclosed technology may be applied, it should be recognized that the illustrated embodiments are only examples and should not be taken as limiting the scope of the disclosure. Rather, the scope of the disclosure is limited by the claims.

## Claims

1. An apparatus for inactivating pathogens in ex vivo stored plasma and/or platelets comprising:
a treatment chamber containing at least one sealed container storing plasma and/or platelets without an added photosensitizing agent present in the plasma and/or platelets;
a light source operable to emit light that is directed to the plasma and/or platelets storage container in the treatment chamber, the emitted light having a wavelength and intensity sufficient to penetrate through a wall of the container and into the plasma and/or platelets, such that the irradiance of the light reaching the plasma and/or platelets inside the wall of the container is sufficient to inactivate pathogens in the plasma and/or platelets without the presence of an added photosensitizing agent in the plasma and/or platelets and without a detrimental effect on the plasma and/or platelets such that the plasma and/or platelets is suitable for future medical use,
wherein the light emitted by the light source has a wavelength spectrum centered on or around 405nm, the center wavelength being the wavelength having the greatest spectral irradiance.

2. The apparatus of claim 1, wherein the light reaching the plasma and/or platelets has a wavelength and intensity sufficient to inactivate bacteria, viruses, and fungi present in an ex vivo plasma and/or platelets stored in a sealed container.

3. The apparatus of any one of claims 1-2, further comprising an agitation mechanism positioned within or coupled to the treatment chamber and configured to agitate the plasma and/or platelets stored in a container within the treatment chamber while the plasma and/or platelets is irradiated with light,
particularly wherein the agitation mechanism comprises a oscillating or vibrating tray on which a blood product storage container rests during treatment.

4. The apparatus of claim 3, wherein the apparatus comprises at least two independent agitation mechanisms,
and/or wherein the agitation mechanism is operable to oscillate the plasma and/or platelets storage container at from 30 to 100 reciprocations per minute during treatment.

5. The apparatus of any one of claims 1-2, further comprising a control system operable to vary the intensity of light emitted from the light source during treatment,
and/or wherein the treatment chamber comprises a hanging mechanism for holding plasma and/or platelets storage containers in a suspended or hanging orientation during treatment,
and/or further comprising a heat sink or a cooling fan arranged to dissipate heat produced by the light source during treatment.

6. The apparatus of any one of claims 1-5, wherein the light source comprises at least a first light source and a second light source positioned on opposing walls of the treatment chamber, the first light source being positioned to direct light at a first surface of the plasma and/or platelets storage container, and the second light source being configured to direct light at a second surface of the plasma and/or platelets storage container that is opposite from the first surface,
and/or wherein the apparatus comprises a temperature control system operable to maintain the air temperature within the treatment chamber within a predetermined range during treatment,
and/or wherein walls of the treatment chamber comprise reflective surfaces configured to reflect light having wavelengths between 380 nm and 500 nm,
and/or further comprising a lens configured to focus or direct light from the light source to a particular region of the treatment chamber,
and/or further comprising a diffuser configured to diffuse light from the light source or blend light from more than one light source.

7. The apparatus of any one of claims 1-6, wherein the light source is operable to emit light with an intensity low enough that light reaching the plasma and/or platelets has an irradiance of 3 mW/cm² or less,
or wherein the light source is operable to emit light with an intensity high enough that light reaching the plasma and/or platelets has an irradiance of 80 mW/cm² or greater,
or wherein the light source is operable to emit light with an intensity such that light reaching a blood has an irradiance in a range from 3 mW/cm² to 80 mW/cm².

8. A method for inactivating pathogens in ex vivo stored plasma and/or platelets, comprising:
providing a sealed storage container containing an ex vivo plasma and/or platelets and without an added photosensitizing agent present in the plasma and/or platelets, the storage container comprising at least one wall that is at least partially transmissive to light having wavelengths from 380 nm to 500 nm; and
irradiating at least one wall of the sealed storage container with light of sufficient intensity and wavelength to penetrate through the at least one wall of the sealed storage container and enter into the plasma and/or platelets, and doing so for sufficient duration to inactivate pathogens in the plasma and/or platelets without causing a substantial detrimental effect on the plasma and/or platelets such that the plasma and/or platelets is suitable for future medical use, wherein the light emitted by the light source has a wavelength spectrum centered on or around 405nm, the center wavelength being the wavelength having the greatest spectral irradiance.

9. The method of claim 8 wherein storage container is plasma and/or platelets transfusion bag,
and/or wherein the storage container comprises PVC.

10. The method of any one of claims 8-9, wherein the light reaching the plasma and/or platelets has an irradiance of from 3 mW/cm² to 80 mW/cm²,
or wherein the light reaching the plasma and/or platelets has an irradiance of 3 mW/cm²
or less, or wherein the light reaching the plasma and/or platelets has an irradiance of 80 mW/cm² or
greater.

11. The method of any one of claims 8-10, further comprising agitating the storage container during the irradiation such that the contained plasma and/or platelets moves within the storage container,
particularly wherein agitating comprising causing the storage container to reciprocate at from 30 to 100 reciprocations per minute.

12. The method of any one of claims 8-11, wherein the irradiating comprises adjusting the intensity of the light emitted by the light source during the treatment,
and/or wherein the irradiating comprises irradiating the storage container from at least two different directions simultaneously,
and/or further comprising circulating air around the storage container during the irradiating to maintain a desired temperature range for the storage container and the plasma and/or platelets,
and/or wherein the irradiating has a duration of at least one hour, and/or
wherein the irradiating has a duration of at least two hours.

13. Use of light having a wavelength of between 380 nm and 500 nm for deactivating pathogens in stored plasma and/or platelets or deactivating virus contamination in stored plasma and/or platelets, without the use of an added photosensitizing agent, wherein the light has a wavelength spectrum centered on or around 405nm, the center wavelength being the wavelength having the greatest spectral irradiance.

## Patentansprüche

1. Einrichtung zum Inaktivieren von Pathogenen in ex-vivo aufbewahrtem Plasma und/oder ex-vivo aufbewahrten Thrombozyten, umfassend:
eine Behandlungskammer, die mindestens einen abgedichteten Behälter enthält, in dem Plasma und/oder Thrombozyten aufbewahrt werden, ohne dass ein hinzugefügtes photosensibilisierendes Mittel in dem Plasma und/oder den Thrombozyten vorhanden ist;
eine Lichtquelle, die betreibbar ist, um Licht zu emittieren, das auf den Plasma- und/oder Thrombozyten-Aufbewahrungsbehälter in der Behandlungskammer gerichtet ist, wobei das emittierte Licht eine Wellenlänge und Intensität aufweist, die ausreichen, um durch eine Wand des Behälters und in das Plasma und/oder die Thrombozyten einzudringen, sodass die Bestrahlungsstärke des Lichts, das das Plasma und/oder die Thrombozyten im Inneren der Wand des Behälters erreicht, ausreicht, um Pathogene in dem Plasma und/oder den Thrombozyten ohne das Vorhandensein eines hinzugefügten photosensibilisierenden Mittels in dem Plasma und/oder den Thrombozyten und ohne eine nachteilige Wirkung auf das Plasma und/oder die Thrombozyten zu inaktivieren, sodass das Plasma und/oder die Thrombozyten für eine zukünftige medizinische Verwendung geeignet ist/sind,
wobei das von der Lichtquelle emittierte Licht ein Wellenlängenspektrum aufweist, das auf oder um 405 nm zentriert ist, wobei die Zentrumswellenlänge die Wellenlänge ist, die die größte spektrale Bestrahlungsstärke aufweist.

2. Vorrichtung nach Anspruch 1, wobei das Licht, das das Plasma und/oder die Thrombozyten erreicht, eine Wellenlänge und Intensität aufweist, die ausreichen, um Bakterien, Viren und Pilze zu inaktivieren, die in ex-vivo-Plasma und/oder - Thrombozyten vorhanden sind, die in einem abgedichteten Behälter aufbewahrt sind.

3. Vorrichtung nach einem der Ansprüche 1 bis 2, ferner umfassend einen Schüttelmechanismus, der innerhalb der Behandlungskammer positioniert oder an diese gekoppelt ist und konfiguriert ist, um das Plasma und/oder die Thrombozyten, die in einem Behälter innerhalb der Behandlungskammer aufbewahrt sind, zu schütteln, während das Plasma und/oder die Thrombozyten mit Licht bestrahlt werden,
wobei der Schüttelmechanismus insbesondere eine oszillierende oder vibrierende Ablage umfasst, auf dem ein Blutprodukt-Aufbewahrungsbehälter während der Behandlung ruht.

4. Einrichtung nach Anspruch 3, wobei die Einrichtung mindestens zwei unabhängige Schüttelmechanismen umfasst,
und/oder wobei der Schüttelmechanismus betreibbar ist, um den Plasma- und/oder Thrombozyten-Aufbewahrungsbehälter während der Behandlung mit 30 bis 100 Hin- und Herbewegungen pro Minute oszillieren zu lassen.

5. Einrichtung nach einem der Ansprüche 1 bis 2, ferner umfassend ein Steuersystem, das betreibbar ist, um die Intensität des von der Lichtquelle emittierten Lichts während der Behandlung zu variieren,
und/oder wobei die Behandlungskammer einen Aufhängemechanismus zum Halten von Plasma- und/oder Thrombozyten-Aufbewahrungsbehältern in einer schwebenden oder hängenden Ausrichtung während der Behandlung umfasst,
und/oder ferner umfassend einen Wärmeableiter oder ein Kühlgebläse, die dazu angeordnet sind, Wärme abzuführen, die von der Lichtquelle während der Behandlung erzeugt wird.

6. Einrichtung nach einem der Ansprüche 1 bis 5, wobei die Lichtquelle mindestens eine erste Lichtquelle und eine zweite Lichtquelle umfasst, die an gegenüberliegenden Wänden der Behandlungskammer positioniert sind, wobei die erste Lichtquelle positioniert ist, um Licht auf eine erste Oberfläche des Plasma- und/oder Thrombozyten-Aufbewahrungsbehälters zu richten, und die zweite Lichtquelle konfiguriert ist, um Licht auf eine zweite Oberfläche des Plasma- und/oder Thrombozyten-Aufbewahrungsbehälters zu richten, die der ersten Oberfläche gegenüberliegt,
und/oder wobei die Einrichtung ein Temperatursteuersystem umfasst, das betreibbar ist, um die Lufttemperatur innerhalb der Behandlungskammer während der Behandlung innerhalb eines vorbestimmten Bereichs aufrechtzuerhalten,
und/oder wobei Wände der Behandlungskammer reflektierende Oberflächen umfassen, die konfiguriert sind, um Licht, das Wellenlängen zwischen 380 nm und 500 nm aufweist, zu reflektieren,
und/oder ferner umfassend eine Linse, die konfiguriert ist, um Licht von der Lichtquelle auf einen jeweiligen Bereich der Behandlungskammer zu fokussieren oder zu richten,
und/oder ferner umfassend einen Diffusor, der konfiguriert ist, um Licht von der Lichtquelle zu diffundieren oder Licht von mehr als einer Lichtquelle zu kombinieren.

7. Einrichtung nach einem der Ansprüche 1 bis 6, wobei die Lichtquelle betreibbar ist, um Licht mit einer Intensität zu emittieren, die niedrig genug ist, dass Licht, das das Plasma und/oder die Thrombozyten erreicht, eine Bestrahlungsstärke von 3 mW/cm² oder weniger aufweist,
oder wobei die Lichtquelle betreibbar ist, um Licht mit einer Intensität zu emittieren, die hoch genug ist, dass Licht, das das Plasma und/oder die Thrombozyten erreicht, eine Bestrahlungsstärke von 80 mW/cm² oder mehr aufweist,
oder wobei die Lichtquelle betreibbar ist, um Licht mit einer solchen Intensität abzugeben, dass Licht, das ein Blut erreicht, eine Bestrahlungsstärke in einem Bereich von 3 mW/cm² bis 80 mW/cm² aufweist.

8. Verfahren zum Inaktivieren von Pathogenen in ex-vivo aufbewahrtem Plasma und/oder ex-vivo aufbewahrten Thrombozyten, umfassend:
Bereitstellen eines abgedichteten Aufbewahrungsbehälters, der ex-vivo-Plasma und/oder -Thrombozyten enthält und ohne dass ein hinzugefügtes photosensibilisierendes Mittel in dem Plasma und/oder den Thrombozyten vorhanden ist, wobei der Aufbewahrungsbehälter mindestens eine Wand umfasst, die mindestens teilweise durchlässig für Licht ist, das Wellenlängen von 380 nm bis 500 nm aufweist; und
Bestrahlen mindestens einer Wand des abgedichteten Aufbewahrungsbehälters mit Licht von ausreichender Intensität und Wellenlänge, um durch die mindestens eine Wand des abgedichteten Aufbewahrungsbehälters einzudringen und in das Plasma und/oder in die Thrombozyten einzutreten, und dies für eine ausreichende Dauer, um Pathogene in dem Plasma und/oder den Thrombozyten zu inaktivieren, ohne eine wesentliche nachteilige Wirkung auf das Plasma und/oder die Thrombozyten zu verursachen, sodass das Plasma und/oder die Thrombozyten für eine zukünftige medizinische Verwendung geeignet ist/sind, wobei das von der Lichtquelle emittierte Licht ein Wellenlängenspektrum aufweist, das bei oder um 405 nm zentriert ist, wobei die Zentrumswellenlänge die Wellenlänge ist, die die größte spektrale Bestrahlungsstärke aufweist.

9. Verfahren nach Anspruch 8, wobei der Aufbewahrungsbehälter ein Plasma- und/oder Thrombozytentransfusionsbeutel ist,
und/oder wobei der Aufbewahrungsbehälter PVC umfasst.

10. Verfahren nach einem der Ansprüche 8 bis 9, wobei das Licht, das das Plasma und/oder die Thrombozyten erreicht, eine Bestrahlungsstärke von 3 mW/cm² bis 80 mW/cm² aufweist,
oder wobei das Licht, das das Plasma und/oder die Thrombozyten erreicht, eine Bestrahlungsstärke von 3 mW/cm² oder weniger aufweist, oder wobei das Licht, das das Plasma und/oder die Thrombozyten erreicht, eine Bestrahlungsstärke von 80 mW/cm² oder
mehr aufweist.

11. Verfahren nach einem der Ansprüche 8 bis 10, ferner umfassend Schütteln des Aufbewahrungsbehälters während der Bestrahlung, sodass sich das enthaltene Plasma und/oder die enthaltenen Thrombozyten innerhalb des Aufbewahrungsbehälters bewegen,
wobei das Schütteln insbesondere das Verursachen umfasst, dass sich der Aufbewahrungsbehälter mit 30 bis 100 Hin- und Herbewegungen pro Minute hin- und herbewegt.

12. Verfahren nach einem der Ansprüche 8 bis 11, wobei das Bestrahlen das Einstellen der Intensität des von der Lichtquelle emittierten Lichts während der Behandlung umfasst,
und/oder wobei das Bestrahlen das Bestrahlen des Aufbewahrungsbehälters aus mindestens zwei unterschiedlichen Richtungen gleichzeitig umfasst,
und/oder ferner umfassend Zirkulieren von Luft um den Aufbewahrungsbehälter herum während des Bestrahlens, um einen gewünschten Temperaturbereich für den Aufbewahrungsbehälter und das Plasma und/oder die Thrombozyten aufrechtzuerhalten,
und/oder wobei das Bestrahlen eine Dauer von mindestens einer Stunde aufweist, und/oder
wobei das Bestrahlen eine Dauer von mindestens zwei Stunden aufweist.

13. Verwendung von Licht, das eine Wellenlänge zwischen 380 nm und 500 nm aufweist, zum Deaktivieren von Pathogenen in aufbewahrtem Plasma und/oder aufbewahrten Thrombozyten oder zum Deaktivieren von Viruskontamination in aufbewahrtem Plasma und/oder aufbewahrten Thrombozyten, ohne die Verwendung eines zugefügten photosensibilisierenden Mittels, wobei das Licht ein Wellenlängenspektrum aufweist, das bei oder um 405 nm zentriert ist, wobei die Zentrumswellenlänge die Wellenlänge ist, die die größte spektrale Bestrahlungsstärke aufweist.

## Revendications

1. Appareil permettant d'inactiver des agents pathogènes dans du plasma et/ou des plaquettes stockés ex vivo, comprenant :
une chambre de traitement contenant au moins un récipient scellé stockant du plasma et/ou des plaquettes sans agent photosensibilisant ajouté présent dans le plasma et/ou les plaquettes ;
une source de lumière permettant d'émettre une lumière qui est dirigée vers le récipient de stockage de plasma et/ou de plaquettes dans la chambre de traitement, la lumière émise ayant une longueur d'onde et une intensité suffisantes pour pénétrer à travers une paroi du récipient et dans le plasma et/ou les plaquettes, de telle sorte que l'irradiance de la lumière atteignant le plasma et/ou les plaquettes à l'intérieur de la paroi du récipient est suffisant pour inactiver les agents pathogènes dans le plasma et/ou les plaquettes sans la présence d'un agent photosensibilisant ajouté dans le plasma et/ou les plaquettes et sans effet préjudiciable sur le plasma et/ou les plaquettes de telle sorte que le plasma et/ou les plaquettes soient appropriés pour un usage médical futur,
dans lequel la lumière émise par la source de lumière a un spectre de longueurs d'onde centré sur ou autour de 405 nm, la longueur d'onde centrale étant la longueur d'onde ayant la plus grande irradiance spectrale.

2. Appareil selon la revendication 1, dans lequel la lumière atteignant le plasma et/ou les plaquettes a une longueur d'onde et une intensité suffisantes pour inactiver les bactéries, les virus et les champignons présents dans un plasma et/ou des plaquettes ex vivo stockés dans un récipient scellé.

3. Appareil selon l'une quelconque des revendications 1-2, comprenant en outre un mécanisme d'agitation positionné à l'intérieur de la chambre de traitement ou accouplé à celle-ci et conçu pour agiter le plasma et/ou les plaquettes stockés dans un récipient à l'intérieur de la chambre de traitement pendant que le plasma et/ou les plaquettes sont irradiés par de la lumière,
en particulier dans lequel le mécanisme d'agitation comprend un plateau oscillant ou vibrant sur lequel repose un récipient de stockage de produit sanguin pendant le traitement.

4. Appareil selon la revendication 3, dans lequel l'appareil comprend au moins deux mécanismes d'agitation indépendants,
et/ou dans lequel le mécanisme d'agitation permet d'osciller le récipient de stockage de plasma et/ou de plaquettes à raison de 30 à 100 mouvements de va-et-vient par minute pendant le traitement.

5. Appareil selon l'une quelconque des revendications 1-2, comprenant en outre un système de commande permettant de varier l'intensité de la lumière émise par la source de lumière pendant le traitement,
et/ou dans lequel la chambre de traitement comprend un mécanisme de suspension pour maintenir des récipients de stockage de plasma et/ou de plaquettes dans une orientation suspendue ou accrochée pendant le traitement,
et/ou comprenant en outre un dissipateur thermique ou un ventilateur de refroidissement agencé pour dissiper la chaleur produite par la source de lumière pendant le traitement.

6. Appareil selon l'une quelconque des revendications 1-5, dans lequel la source de lumière comprend au moins une première source de lumière et une seconde source de lumière positionnées sur des parois opposées de la chambre de traitement, la première source de lumière étant positionnée pour diriger la lumière vers une première surface du récipient de stockage de plasma et/ou de plaquettes, et la seconde source de lumière étant configurée pour diriger la lumière vers une seconde surface du récipient de stockage de plasma et/ou de plaquettes qui est à l'opposé de la première surface,
et/ou dans lequel l'appareil comprend un système de commande de température permettant de maintenir la température de l'air à l'intérieur de la chambre de traitement dans une plage prédéterminée pendant le traitement,
et/ou dans lequel les parois de la chambre de traitement comprennent des surfaces réfléchissantes conçues pour réfléchir la lumière ayant des longueurs d'onde comprises entre 380 nm et 500 nm,
et/ou comprenant en outre une lentille configurée pour focaliser ou diriger la lumière provenant de la source de lumière vers une région particulière de la chambre de traitement,
et/ou comprenant en outre un diffuseur configuré pour diffuser la lumière provenant de la source de lumière ou mélanger la lumière provenant de plus d'une source de lumière.

7. Appareil selon l'une quelconque des revendications 1-6, dans lequel la source de lumière permet d'émettre de la lumière avec une intensité suffisamment faible pour que la lumière atteignant le plasma et/ou les plaquettes ait une irradiance de 3 mW/cm² ou moins,
ou dans lequel la source de lumière permet d'émettre de la lumière avec une intensité suffisamment élevée pour que la lumière atteignant le plasma et/ou les plaquettes ait une irradiance de 80 mW/cm² ou plus,
ou dans lequel la source de lumière permet d'émettre de la lumière avec une intensité de telle sorte que la lumière atteignant un sang a une irradiance dans une plage de 3 mW/cm² à 80 mW/cm².

8. Procédé d'inactivation d'agents pathogènes dans du plasma et/ou des plaquettes stockés ex vivo, comprenant :
la fourniture d'un récipient de stockage scellé contenant un plasma et/ou des plaquettes ex vivo et sans agent photosensibilisant ajouté présent dans le plasma et/ou les plaquettes, le récipient de stockage comprenant au moins une paroi qui est au moins partiellement transmissive à la lumière ayant des longueurs d'onde de 380 nm à 500 nm ; et
l'irradiation d'au moins une paroi du récipient de stockage scellé avec une lumière d'une intensité et d'une longueur d'onde suffisantes pour pénétrer à travers l'au moins une paroi du récipient de stockage scellé et entrer dans le plasma et/ou les plaquettes, et ce pendant une durée suffisante pour inactiver les agents pathogènes dans le plasma et/ou les plaquettes sans causer d'effet préjudiciable substantiel sur le plasma et/ou les plaquettes de telle sorte que le plasma et/ou les plaquettes soient appropriés pour un usage médical futur, dans lequel la lumière émise par la source de lumière a un spectre de longueurs d'onde centré sur ou autour de 405 nm, la longueur d'onde centrale étant la longueur d'onde ayant la plus grande irradiance spectrale.

9. Procédé selon la revendication 8, dans lequel le récipient de stockage est une poche de transfusion de plasma et/ou de plaquettes,
et/ou dans lequel le récipient de stockage comprend du PVC.

10. Procédé selon l'une quelconque des revendications 8-9, dans lequel la lumière atteignant le plasma et/ou les plaquettes a une irradiance de 3 mW/cm² à 80 mW/cm²,
ou dans lequel la lumière atteignant le plasma et/ou les plaquettes a une irradiance de 3 mW/cm² ou moins, ou dans lequel la lumière atteignant le plasma et/ou les plaquettes a une irradiance de 80 mW/cm² ou
supérieure.

11. Procédé selon l'une quelconque des revendications 8-10, comprenant en outre l'agitation du récipient de stockage pendant l'irradiation de telle sorte que le plasma et/ou les plaquettes contenus se déplacent à l'intérieur du récipient de stockage,
en particulier dans lequel l'agitation comprend le fait d'amener le récipient de stockage à effectuer un mouvement de va-et-vient à raison de 30 à 100 mouvements de va-et-vient par minute.

12. Procédé selon l'une quelconque des revendications 8-11, dans lequel l'irradiation comprend l'ajustement de l'intensité de la lumière émise par la source de lumière pendant le traitement,
et/ou dans lequel l'irradiation comprend l'irradiation du récipient de stockage à partir d'au moins deux directions différentes simultanément,
et/ou comprenant en outre la circulation d'air autour du récipient de stockage pendant l'irradiation pour maintenir une plage de températures souhaitée pour le récipient de stockage et le plasma et/ou les plaquettes,
et/ou dans lequel l'irradiation a une durée d'au moins une heure, et/ou
dans lequel l'irradiation a une durée d'au moins deux heures.

13. Utilisation d'une lumière ayant une longueur d'onde comprise entre 380 nm et 500 nm pour désactiver des agents pathogènes dans du plasma et/ou des plaquettes stockés ou désactiver une contamination virale dans du plasma et/ou des plaquettes stockés, sans l'utilisation d'un agent photosensibilisant ajouté, dans laquelle la lumière a un spectre de longueurs d'onde centré sur ou autour de 405 nm, la longueur d'onde centrale étant la longueur d'onde ayant la plus grande irradiance spectrale.
